# EUROPÄISCHE PATENTSCHRIFT

(11) **EP 1 599 179 B1**
(45) Veröffentlichungstag und Bekanntmachung des Hinweises auf die Patenterteilung: **11.10.2006**
(21) Anmeldenummer: 04707512.2
(22) Anmeldetag: 03.02.2004
(51) Int. Cl.: A61K 8/23, A61K 8/49, A61Q 5/10

(54) **MITTEL UND VERFAHREN ZUM FÄRBEN VON KERATINFASERN**
AGENT AND METHOD FOR DYEING KERATIN FIBRES
AGENT ET PROCEDE DE COLORATION DE FIBRES KERATINIQUES

(30) Priorität: 05.03.2003 DE 10309523
(43) Veröffentlichungstag der Anmeldung: 30.11.2005
(73) Patentinhaber: Wella Aktiengesellschaft, 64274 Darmstadt (DE)
(72) Erfinder: PASQUIER, Cécile, CH-1723 Marly (CH); BUCLIN, Véronique, CH-1638 Morlon (CH); KIENER, Caroline, CH-1723 Marly (CH); ROULIN, Anita, CH-1695 Villarlod (CH); BRAUN, Hans-Jürgen, CH-3182 Ueberstorf (CH)
(86) Internationale Anmeldenummer: PCT/EP2004/000961
(87) Internationale Veröffentlichungsnummer: WO 2004/078153

(56) Entgegenhaltungen:
- EP-A- 0 664 114
- DE-A- 2 334 738
- DE-A- 10 155 907
- US-A- 3 634 013

## Beschreibung

Gegenstand der vorliegenden Anmeldung ist ein Mittel zur Färbung von Keratinfasern, wie zum Beispiel Seide, Wolle oder Haaren und insbesondere menschlichen Haaren, auf Basis von Oxidationsfarbstoffen, welches mindestens ein heterozyklisches Hydrazon-Derivat (der Thiazol- oder Oxazol-Familie) als Entwickler und mindestens ein Persulfatsalz als Oxidationsmittel enthält, ein Mehrkomponenten-Kit sowie ein Verfahren zum Färben von Keratinfasern unter Verwendung dieses Mittels.

Haarfärbemittel werden je nach zu färbender Ausgangshaarfarbe und gewünschtem Endresultat hauptsächlich in die Gruppe der Oxidationsfärbemittel oder der Tönungen unterteilt. Oxidationsfärbemittel eignen sich hervorragend für die Abdeckung von höheren Grauanteilen, hierbei werden die bei einem Grauanteil von bis zu 50 % verwendeten Oxidationsfärbemittel in der Regel als oxidative Tönungen bezeichnet, während die bei einem Grauanteil von über 50 % oder zum "Hellerfärben" verwendeten Oxidationsfärbemittel in der Regel als sogenannte oxidative Farben bezeichnet werden. Direktziehende Farbstoffe sind hauptsächlich in nicht-oxidativen Färbemitteln (sogenannten Tönungsmitteln) enthalten. Einige direktziehende Farbstoffe, wie zum Beispiel Nitrofarbstoffe, können aufgrund ihrer geringen Größe in das Haar eindringen und es -zumindest in den äusseren Bereichen- direkt anfärben. Derartige Tönungen sind sehr haarschonend und überstehen in der Regel 6 bis 8 Haarwäschen. Direktziehende Farbstoffe werden ebenfalls oft in oxidativen Färbemitteln zur Erzeugung bestimmter Nuancen beziehungsweise zur Intensivierung der Farbe eingesetzt. Die bisher bekannten Färbesysteme können jedoch die an Färbemittel gestellten Anforderungen nicht in jeder Hinsicht, besonders im Hinblick auf Glanz und Intensität der Färbungen, erfüllen.

Aus der US-A 3634013 sind Färbemittel für Fasern bekannt, welche ein heterozyklisches Hydrazonderivat, eine Kupplersubstanz und als Oxidationsmittel Wasserstoffperoxid oder dessen Additionsverbindungen enthalten; während aus der DE-A 2334738 oxidative Haarfärbemittel bekannt sind, welche eine Kombination aus N-Methyl-benzthiazolon-2-hydrazon und einem 4-Hydroxy-chinolon-2-derivat enthalten, wobei als Oxidationsmittel Wasserstoffperoxid verwendet wird. Die EP-A 0664114 offenbart Catechole und Persulfate enthaltende Haarfärbemittel.

Überraschenderweise wurde nunmehr gefunden, daß heterozyklische Hydrazone der Thiazol- oder Oxazol-Familie mit üblichen Kupplersubstanzen, wie zum Beispiel aromatische Hydroxyl- und/ oder Aminogruppen enthaltenden Verbindungen, in Gegenwart von Persulfatsalzen intensive Färbungen im gelben bis blauen Farbbereich ermöglichen.
Dieses neue Färbemittel ergibt besonders brillante und intensive Färbungen, die eine sehr hohe Schweissbeständigkeit zeigen.

Gegenstand der vorliegenden Erfindung ist daher ein Mittel zur Färbung von Keratinfasern (A), wie zum Beispiel Wolle, Seide oder Haaren und insbesondere menschlichen Haaren, welches dadurch gekennzeichnet ist, dass es (a) mindestens ein Hydrazon-Derivat der Formel (I) oder dessen physiologisch verträgliches Salz, 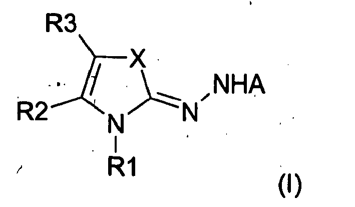 worin **X** gleich Sauerstoff oder Schwefel ist;
**A** Wasserstoff, eine Acetylgruppe, eine Trifluoracetylgruppe, eine Formylgruppe, eine (C₁-C₆)-Alkylsulfonylgruppe oder eine Arylsulfonylgruppe darstellt;
**R1** gleich einer gesättigten oder ungesättigten (C₁-C₁₂)-Alkylgruppe, einer mit einem Halogenatom (F, Cl, Br, J) substituierten (C₁-C₁₂)-Alkylgruppe, einer Hydroxy-(C₁-C₁₂)-alkylgruppe, einer Amino-(C₁-C₁₂)-alkylgruppe, einer Sulfonsäure-(C₁-C₁₂)-alkyl-gruppe, einer Formylgruppe, einer C(O)-(C₁-C₁₂)-Alkylgruppe, einer C(O)-Phenylgruppe, einer C(O)NH-(C₁₋C₁₂)-Alkylgruppe, einer C(O)NH-Phenylgruppe, einer substituierten oder unsubstituierten Phenylgruppe, oder einer Benzylgruppe ist;
**R2** und **R3** gleich oder verschieden sein können und unabhängig voneinander Wasserstoff, ein Halogenatom (F, Cl, Br, J), eine gesättigte oder ungesättigte (C₁-C₁₂)-Alkylgruppe, eine mit einem Halogenatom (F, Cl, Br, J) substituierte (C₁-C₁₂)-Alkylgruppe, eine Hydroxy-(C₁-C₁₂)-alkylgruppe, eine (C₁-C₁₂)-Alkoxygruppe, eine Cyanogruppe, eine Nitrogruppe, eine Aminogruppe, eine (C₁-C₁₂)-Alkylaminogruppe, eine (C₁-C₁₂)-Dialkylaminogruppe, eine Carbonsäure, eine C(O)O-(C₁-C₁₂)-Alkylgruppe, eine substituierte oder unsubstituierte C(O)O-Phenylgruppe, eine sub-stituierte oder unsubstituierte Phenylgruppe oder eine Naphthylgruppe darstellen, oder **R2** und **R3** gemeinsam mit dem Restmolekül ein heterozyklisches oder carbozyklisches, gesättigtes oder ungesättigtes, substituiertes oder unsubstituiertes Ringsystem bilden;
(b) mindestens eine an sich bekannten Kupplersubstanz oder deren physiologisch verträgliches Salz,
sowie (c) als oxidatives Mittel ein Persulfatsalz enthält.

Je nach dem pH-Wert des Mittels kann die Verbindung der Formel (I) auch im Gleichgewicht mit der Verbindung der Formel (I') vorliegen:

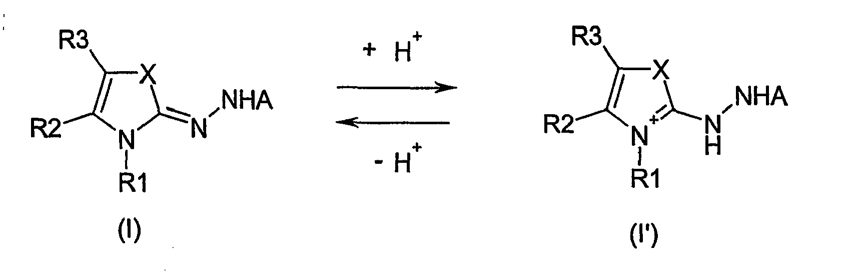

Bevorzugt sind Hydrazon-Derivate der Formel (I) oder deren physiologisch verträgliche Salze, worin **X** gleich Schwefel ist und **A** Wasserstoff darstellt. Hydrazon-Derivate der Formel (I) oder deren physiologisch verträgliche Salze, bei denen **X** gleich Schwefel ist, **A** gleich Wasserstoff ist, **R1** gleich einer gesättigten oder ungesättigten (C₁-C₁₂)-Alkylgruppe, einer Hydroxy-(C₁-C₁₂)-alkylgruppe, einer Amino-(C₁-C₁₂)-alkylgruppe, oder einer substituierten oder unsubstituierten Phenylgruppe ist, und **R2** und **R3** unabhängig voneinander Wasserstoff, eine gesättigte oder ungesättigte (C₁-C₁₂)-Alkylgruppe, eine Cyanogruppe, eine Nitrogruppe, eine Aminogruppe, eine (C₁-C₁₂)-Alkylaminogruppe, eine (C₁-C₁₂)-Dialkylaminogruppe, eine C(O)O-(C₁-C₁₂)-Alkylgruppe oder eine substituierte oder unsubstituierte Phenylgruppe oder eine Naphtylgruppe darstellen, oder **R2** und **R3** gemeinsam mit dem Restmolekül ein carbo-zyklisches, ungesättigtes, substituiertes oder unsubstituiertes Ringsystem bilden, sind besonders bevorzugt.

Als Beispiel für die Verbindungen der Formel (I) können die folgenden Verbindungen gennant werden:
3-Methyl-2(3H)-thiazolon-hydrazon,
3,4-Dimethyl-2(3H)-thiazolon-hydrazon,
4-tert-Butyl-3-methyl-2(3H)-thiazolon-hydrazon,
3-Methyl-4-phenyl-2(3H)-thiazolon-hydrazon,
3-Methyl-4-(4-tolyl)-2(3H)-thiazolon-hydrazon,
4-(4-Methoxy)phenyl-3-methyl-2(3H)-thiazolon-hydrazon,
4-(4-Ethoxy)phenyl-3-methyl-2(3H)-thiazolon-hydrazon,
4-(4-Bromphenyl)-3-methyl-2(3H)-thiazolon-hydrazon,
4-(3-Bromphenyl)-3-methyl-2(3H)-thiazolon-hydrazon,
4-(4-Chlorphenyl)-3-methyl-2(3H)-thiazolon-hydrazon,
4-(3-Chlorphenyl)-3-methyl-2(3H)-thiazolon-hydrazon,
3-Methyl-4-(4-nitrophenyl)-2(3H)-thiazolon-hydrazon,
3-Methyl-4-(3-nitrophenyl)-2(3H)-thiazolon-hydrazon,
4-([1,1'-Biphenyl]-4-yl)-3-methyl-2(3H)-thiazolon-hydrazon,
3-Methyl-4-(2-naphthalenyl)-2(3H)-thiazolon-hydrazon,
2-Hydrazono-2,3-dihydro-3-methyl-4-thiazolcarbonsäureethylester,
3,4,5-Trimethyl-2(3H)-thiazolon-hydrazon,
3,4-Dimethyl-5-phenyl-2(3H)-thiazolon-hydrazon,
3,5-Dimethyl-4-phenyl-2(3H)-thiazolon-hydrazon,
3-Methyl-4,5-diphenyl-2(3H)-thiazolon-hydrazon,
5-Ethyl-3-methyl-4-phenyl-2(3H)-thiazolon-hydrazon,
4-(4-Bromphenyl)-3-methyl-5-phenyl-2(3H)-thiazolon-hydrazon,
3-Methyl-5-phenyl-4-(4-tolyl)-2(3H)-thiazolon-hydrazon,
5-(4-Chlorphenyl)-4-phenyl-3-methyl-2(3H)-thiazolon-hydrazon,
5-(4-Chlorphenyl)-4-(4-methoxyphenyl)-3-methyl-2(3H)-thiazolon-hydrazon,
2-Hydrazono-2,3-dihydro-3,4-dimethyl-4-thiazolcarbonsäureethylester,
4-Amino-2-hydrazono-2,3-dihydro-3-methyl-5-thiazolcarbonitril,
3-Ethyl-4,5-dimethyl-2(3H)-thiazolon-hydrazon,
2-Hydrazono-2,3-dihydro-3-ethyl-4-methyl-thiazolcarbonsäureethylester,
5-Methyl-3-(1-methylethyl)-4-phenyl-2(3H)-thiazolon-hydrazon,
4,5-Dimethyl-3-(1-methylethyl)- 2(3H)-thiazolon-hydrazon,
3-(1-Methylethyl)-4,5-diphenyl-2(3H)-thiazolon-hydrazon,
4,5-Dimethyl-3-propyl-2(3H)-thiazolon-hydrazon,
4,5-Diphenyl-3-propyl-2(3H)-thiazolon-hydrazon,
3-Butyl-4,5-diphenyl-2(3H)-thiazolon-hydrazon,
4,5-Dimethyl-3-(2-methylpropyl)- 2(3H)-thiazolon-hydrazon,
3-(2-Methylpropyl)- 4,5-diphenyl-2(3H)-thiazolon-hydrazon,
3-Hydroxyethyl-2(3H)-thiazolon-hydrazon,
3-Hydroxyethyl-4-methyl-2(3H)-thiazolon-hydrazon,
3-Hydroxyethyl-4,5-dimethyl-2(3H)-thiazolon-hydrazon,
3-Aminoethyl-2(3H)-thiazolon-hydrazon,
3-Aminoethyl-4-methyl-2(3H)-thiazolon-hydrazon,
3-Aminoethyl-4,5-dimethyl-2(3H)-thiazolon-hydrazon,
3,4-Diphenyl-2(3H)-thiazolon-hydrazon,
4-Methyl-3-phenyl-2(3H)-thiazolon-hydrazon,
4-p-Biphenylyl-3-phenyl-2(3H)-thiazolon-hydrazon,
4-(4-Methoxy)phenyl-3-phenyl-2(3H)-thiazolon-hydrazon,
4-tert-Butyl-3-phenyl-2(3H)-thiazolon-hydrazon,
4,5-Dimethyl-3-phenyl-2(3H)-thiazolon-hydrazon,
5-Methyl-3,4-diphenyl-2(3H)-thiazolon-hydrazon,
3,4,5-Triphenyl-2(3H)-thiazolon-hydrazon,
4,5-Dimethyl-3-(phenylmethyl)-2(3H)-thiazolon-hydrazon,
3-(2-Propenyl)-2(3H)-thiazolon-hydrazon,
4-Methyl-3-(2-propenyl)-2(3H)-thiazolon-hydrazon,
4-tert-Butyl-3-(2-propenyl)-2(3H)-thiazolon-hydrazon,
4-Phenyl-3-(2-propenyl)-2(3H)-thiazolon-hydrazon,
4,5-Dimethyl-3-(2-propenyl)-2(3H)-thiazolon-hydrazon,
4,5-Diphenyl-3-(2-propenyl)-2(3H)-thiazolon-hydrazon,
2-Hydrazono-2,3-dihydro-3-[(phenylamino)carbonyl]-4-methyl-thiazolcarbonsäureethylester,
3-Methyl-4,5,6,7-tetrahydro-2(3H)-benzothiazolon-hydrazon,
3-Methyl-2(3H)-benzothiazolon-hydrazon,
3,6-Dimethyl-2(3H)-benzothiazolon-hydrazon,
6-Chlor-3-methyl-2(3H)-benzothiazolon-hydrazon,
7-Chlor-3-methyl-2(3H)-benzothiazolon-hydrazon,
6-Hydroxy-3-methyl-2(3H)-benzothiazolon-hydrazon,
5-Methoxy-3-methyl-2(3H)-benzothiazolon-hydrazon,
7-Methoxy-3-methyl-2(3H)-benzothiazolon-hydrazon,
5,6-Dimethoxy-3-methyl-2(3H)-benzothiazolon-hydrazon,
5-Ethoxy-3-methyl-2(3H)-benzothiazolon-hydrazon,
6-Ethoxy-3-methyl-2(3H)-benzothiazolon-hydrazon,
3-Methyl-5-nitro-2(3H)-benzothiazolon-hydrazon,
3-Methyl-6-nitro-2(3H)-benzothiazolon-hydrazon,
5-Acetamido-3-methyl-2(3H)-benzothiazolon-hydrazon,
6-Acetamido-3-methyl-2(3H)-benzothiazolon-hydrazon,
5-Anilino-3-methyl-2(3H)-benzothiazolon-hydrazon,
6-Anilino-3-methyl-2(3H)-benzothiazolon-hydrazon,
2-Hydrazono-2,3-dihydro-3-methyl-6-benzothiazol-carbonsäure,
2-Hydrazono-2,3-dihydro-3-methyl-4-benzothiazol-sulfonsäure,
2-Hydrazono-2,3-dihydro-3-methyl-5-benzothiazol-sulfonsäure,
2-Hydrazono-2,3-dihydro-3-methyl-6-benzothiazol-sulfonsäure,
2-Hydrazono-2,3-dihydro-3-methyl-7-benzothiazol-sulfonsäure,
2-Hydrazono-2,3-dihydro-N,N,3-trimethyl-6-benzothiazol-sulfonsäureamid,
[(2-Hydrazono-2,3-dihydro-3-methyl-6-benzothiazolyl)oxy]essigsäure-hydrazid,
3-Methyl-naphtho[2,3-d]thiazol-2(3H)-on-hydrazon,
3-Ethyl-2(3H)-benzothiazolon-hydrazon,
6-Ethoxy-3-ethyl-2(3H)-benzothiazolon-hydrazon,
3-Propyl-2(3H)-benzothiazolon-hydrazon,
3-Butyl-2(3H)-benzothiazolon-hydrazon,
3-Hexyl-2(3H)-benzothiazolon-hydrazon,
3-Hydroxyethyl-2(3H)-benzothiazolon-hydrazon,
3-Aminoethyl-2(3H)-benzothiazolon-hydrazon,
3-p-Methylbenzyl-2(3H)-benzothiazolon-hydrazon,
2-Hydrazono-2,3-dihydro-3-(2-hydroxyethyl)-6-benzothiazol-carbonsäure,
2-Hydrazono-2,3-dihydro-6-methoxy-3(2H)-benzothiazol-propan-sulfonsäure,
6-Hexadecyloxy-2-hydrazono-3(2H)-benzothiazol-propan-sulfonsäure,
2-Oxo-3-benzothiazolin-essigsäureethylester-hydrazon,
3-Acetyl-2(3H)-benzothiazolon-hydrazon,
2-Hydrazono-3(2H)-benzothiazol-carboxaldehyd,
3-Methyl-2(3H)-oxazolon-hydrazon,
3-Phenyl-2(3H)-oxazolon-hydrazon,
3-Methyl-2(3H)-benzoxazolon-hydrazon,
3-Phenyl-2(3H)-benzoxazolon-hydrazon,
N-Acetyl-3-methyl-2(3H)-thiazolon-hydrazon,
N-Acetyl-3,4-dimethyl-2(3H)-thiazolon-hydrazon,
N-Acetyl-3-methyl-4-phenyl-2(3H)-thiazolon-hydrazon,
N-Acetyl-4-(4-methoxy)phenyl-3-methyl-2(3H)-thiazolon-hydrazon,
N-Acetyl-3-methyl-4-(4-nitrophenyl)-2(3H)-thiazolon-hydrazon,
N-Acetyl-4-([1,1'-biphenyl]-4-yl)-3-methyl-2(3H)-thiazolon-hydrazon,
N-Acetyl-3-methyl-4-(2-naphtalenyl)-2(3H)-thiazolon-hydrazon,
N-Acetyl-2-hydrazono-2,3-dihydro-3-methyl-4-thiazolcarbonsäure-ethylester,
N-Acetyl-3,4,5-trimethyl-2(3H)-thiazolon-hydrazon,
N-Acetyl-3,4-dimethyl-5-phenyl-2(3H)-thiazolon-hydrazon,
N-Acetyl-3,5-dimethyl-4-phenyl-2(3H)-thiazolon-hydrazon,
N-Acetyl-3-methyl-4,5-diphenyl-2(3H)-thiazolon-hydrazon,
N-Acetyl-3-ethyl-4,5-dimethyl-2(3H)-thiazolon-hydrazon,
N-Acetyl-4-methyl-3-phenyl-2(3H)-thiazolon-hydrazon,
N-Acetyl-4,5-dimethyl-3-phenyl-2(3H)-thiazolon-hydrazon,
N-Acetyl-3,4-diphenyl-2(3H)-thiazolon-hydrazon,
N-Acetyl-4-p-biphenylyl-3-phenyl-2(3H)-thiazolon-hydrazon,
N-Acetyl-4-(4-methoxy)phenyl-3-phenyl-2(3H)-thiazolon-hydrazon,
N-Acetyl-4-tert-butyl-3-phenyl-2(3H)-thiazolon-hydrazon,
N-Acetyl-3,4,5-triphenyl-2(3H)-thiazolon-hydrazon,
N-Acetyl-3-methyl-2(3H)-benzothiazolon-hydrazon,
N-Acetyl-3-ethyl-2(3H)-benzothiazolon-hydrazon,
N-Acetyl-3-butyl-2(3H)-benzothiazolon-hydrazon,
N-Acetyl-3-hexyl-2(3H)-benzothiazolon-hydrazon,
N-Acetyl-3-p-methylbenzyl-2(3H)-benzothiazolon-hydrazon,
N-Acetyl-3-methyl-2(3H)-oxazolon-hydrazon,
N-Acetyl-3-phenyl-2(3H)-oxazolon-hydrazon,
N-Acetyl-3-methyl-2(3H)-benzoxazolon-hydrazon,
N-Acetyl-3-phenyl-2(3H)-benzoxazolon-hydrazon,
N-Formyl-3-methyl-2(3H)-thiazolon-hydrazon,
N-Formyl-3,4-dimethyl-2(3H)-thiazolon-hydrazon,
N-Formyl-3-methyl-4-phenyl-2(3H)-thiazolon-hydrazon,
N-Formyl-4-(4-methoxy)phenyl-3-methyl-2(3H)-thiazolon-hydrazon,
N-Formyl-3-methyl-4-(4-nitrophenyl)-2(3H)-thiazolon-hydrazon,
N-Formyl-4-([1,1'-biphenyl]-4-yl)-3-methyl-2(3H)-thiazolon-hydrazon,
N-Formyl-3-methyl-4-(2-naphtalenyl)-2(3H)-thiazolon-hydrazon,
N-Formyl-2-hydrazono-2,3-dihydro-3-methyl-4-thiazolcarbonsäure-ethylester,
N-Formyl-3,4,5-trimethyl-2(3H)-thiazolon-hydrazon,
N-Formyl-3,4-dimethyl-5-phenyl-2(3H)-thiazolon-hydrazon,
N-Formyl-3,5-dimethyl-4-phenyl-2(3H)-thiazolon-hydrazon,
N-Formyl-3-methyl-4,5-diphenyl-2(3H)-thiazolon-hydrazon,
N-Formyl-3-ethyl-4,5-dimethyl-2(3H)-thiazolon-hydrazon,
N-Formyl-4-methyl-3-phenyl-2(3H)-thiazolon-hydrazon,
N-Formyl-3,4-diphenyl-2(3H)-thiazolon-hydrazon,
N-Formyl-4-p-biphenylyl-3-phenyl-2(3H)-thiazolon-hydrazon,
N-Formyl-4-(4-methoxy)phenyl-3-phenyl-2(3H)-thiazolon-hydrazon,
N-Formyl-4-tert-butyl-3-phenyl-2(3H)-thiazolon-hydrazon,
N-Formyl-4,5-dimethyl-3-phenyl-2(3H)-thiazolon-hydrazon,
N-Formyl-5-methyl-3,4-diphenyl-2(3H)-thiazolon-hydrazon,
N-Formyl-3,4,5-triphenyl-2(3H)-thiazolon-hydrazon,
N-Formyl-3-methyl-2(3H)-benzothiazolon-hydrazon,
N-Formyl-3-ethyl-2(3H)-benzothiazolon-hydrazon,
N-Formyl-3-butyl-2(3H)-benzothiazolon-hydrazon,
N-Formyl-3-hexyl-2(3H)-benzothiazolon-hydrazon,
N-Formyl-3-p-methylbenzyl-2(3H)-benzothiazolon-hydrazon,
N-Formyl-3-methyl-2(3H)-oxazolon-hydrazon,
N-Formyl-3-phenyl-2(3H)-oxazolon-hydrazon,
N-Formyl-3-methyl-2(3H)-benzoxazolon-hydrazon und
N-Formyl-3-phenyl-2(3H)-benzoxazolon-hydrazon.

Unter den Verbindungen der Formel (I) sind die folgenden Thiazolon-hydrazon-Derivate besonders bevorzugt:
3-Methyl-2(3H)-thiazolon-hydrazon,
3,4-Dimethyl-2(3H)-thiazolon-hydrazon,
4-tert-Butyl-3-methyl-2(3H)-thiazolon-hydrazon,
3-Methyl-4-phenyl-2(3H)-thiazolon-hydrazon,
3-Methyl-4-(4-tolyl)-2(3H)-thiazolon-hydrazon,
4-(4-Methoxy)phenyl-3-methyl-2(3H)-thiazolon-hydrazon,
4-(4-Ethoxyphenyl)-3-methyl-2(3H)-thiazolon-hydrazon,
4-(4-Bromphenyl)-3-methyl-2(3H)-thiazolon-hydrazon,
4-(3-Bromphenyl)-3-methyl-2(3H)-thiazolon-hydrazon,
4-(4-Chlorphenyl)-3-methyl-2(3H)-thiazolon-hydrazon,
4-(3-Chlorphenyl)-3-methyl-2(3H)-thiazolon-hydrazon,
3-Methyl-4-(4-nitrophenyl)-2(3H)-thiazolon-hydrazon,
3-Methyl-4-(3-nitrophenyl)-2(3H)-thiazolon-hydrazon,
4-([1,1'-Biphenyl]-4-yl)-3-methyl-2(3H)-thiazolon-hydrazon,
2-Hydrazono-2,3-dihydro-3-methyl-4-thiazolcarbonsäureethylester,
3,4,5-Trimethyl-2(3H)-thiazolon-hydrazon,
3,4-Dimethyl-5-phenyl-2(3H)-thiazolon-hydrazon,
3,5-Dimethyl-4-phenyl-2(3H)-thiazolon-hydrazon,
3-Methyl-4,5-diphenyl-2(3H)-thiazolon-hydrazon,
5-Ethyl-3-methyl-4-phenyl-2(3H)-thiazolon-hydrazon,
4-(4-Bromphenyl)-3-methyl-5-phenyl-2(3H)-thiazolon-hydrazon,
3-Methyl-5-phenyl-4-(4-tolyl)-2(3H)-thiazolon-hydrazon,
5-(4-Chlorphenyl)-4-phenyl-3-methyl-2(3H)-thiazolon-hydrazon,
5-(4-Chlorphenyl)-4-(4-methoxyphenyl)-3-methyl-2(3H)-thiazolon-hydrazon,
2-Hydrazono-2,3-dihydro-3,4-dimethyl-4-thiazol-carbonsäureethylester,
4-Amino-2-hydrazino-2,3-dihydro-3-methyl-5-thiazol-carbonitril,
3-Ethyl-4,5-dimethyl-2(3H)-thiazolon-hydrazon,
2-Hydrazono-2,3-dihydro-3-ethyl-4-methyl-thiazol-carbonsäureethylester,
5-Methyl-3-(1-methylethyl)-4-phenyl-2(3H)-thiazolon-hydrazon,
3-(1-Methylethyl)-4,5-diphenyl-2(3H)-thiazolon-hydrazon,
4,5-Dimethyl-3-propyl-2(3H)-thiazolon-hydrazon,
4,5-Diphenyl-3-propyl-2(3H)-thiazolon-hydrazon,
3-Butyl-4,5-diphenyl-2(3H)-thiazolon-hydrazon,
4,5-Dimethyl-3-(2-methylpropyl)- 2(3H)-thiazolon-hydrazon,
3-(2-Methylpropyl)- 4,5-diphenyl-2(3H)-thiazolon-hydrazon,
3-(2-Propenyl)-2(3H)-thiazolon-hydrazon,
4-Methyl-3-(2-propenyl)-2(3H)-thiazolon-hydrazon,
4-tert-Butyl-3-(2-propenyl)-2(3H)-thiazolon-hydrazon,
4-Phenyl-3-(2-propenyl)-2(3H)-thiazolon-hydrazon,
4,5-Dimethyl-3-(2-propenyl)-2(3H)-thiazolon-hydrazon,
3-Hydroxyethyl-2(3H)-thiazolon-hydrazon,
3-Hydroxyethyl-4-methyl-2(3H)-thiazolon-hydrazon,
3-Hydroxyethyl-4,5-dimethyl-2(3H)-thiazolon-hydrazon,
3-Aminoethyl-2(3H)-thiazolon-hydrazon,
3-Aminoethyl-4-methyl-2(3H)-thiazolon-hydrazon,
3-Aminoethyl-4,5-dimethyl-2(3H)-thiazolon-hydrazon,
3-Phenyl-2(3H)-thiazolon-hydrazon,
4-Methyl-3-phenyl-2(3H)-thiazolon-hydrazon,
3,4-Diphenyl-2(3H)-thiazolon-hydrazon,
4,5-Dimethyl-3-phenyl-2(3H)-thiazolon-hydrazon,
4-p-Biphenylyl-3-phenyl-2(3H)-thiazolon-hydrazon,
4-(4-Methoxy)phenyl-3-phenyl-2(3H)-thiazolon-hydrazon,
4-tert-Butyl-3-phenyl-2(3H)-thiazolon-hydrazon,
5-Methyl-3,4-diphenyl-2(3H)-thiazolon-hydrazon,
3,4,5-Triphenyl-2(3H)-thiazolon-hydrazon,
3-Methyl-4,5,6,7-tetrahydro-2(3H)-benzothiazolon-hydrazon,
3-Methyl-2(3H)-benzothiazolon-hydrazon,
3-Ethyl-2(3H)-benzothiazolon-hydrazon,
3-Butyl-2(3H)-benzothiazolon-hydrazon,
3-Hexyl-2(3H)-benzothiazolon-hydrazon,
3-Hydroxyethyl-2(3H)-benzothiazolon-hydrazon,
3-Aminoethyl-2(3H)-benzothiazolon-hydrazon.

Die Verbindungen der Formel (I) sind zum Teil im Handel erhältlich. Sie können jedoch auch nach aus der Literatur bekannten Syntheseverfahren, beispielsweise der Vorschrift in Research Disclosure October 1978, Seite 42 - 44, No. 17434, oder in Analogie zu den in der DE 1049381 B beschriebenen Verfahren hergestellt werden.

Als Kupplersubstanzen kommen insbesondere die folgenden Kupplersubstanzen oder deren Salze in Betracht:
N-(3-Dimethylamino-phenyl)-harnstoff, 2,6-Diamino-pyridin, 2-Amino-4-[(2-hydroxyethyl)amino]-anisol, 2,4-Diamino-1-fluor-5-methyl-benzol, 2,4-Diamino-1-methoxy-5-methyl-benzol, 2,4-Diamino-1-ethoxy-5-methylbenzol, 2,4-Diamino-1-(2-hydroxy-ethoxy)-5-methyl-benzol, 2,4-Di[(2-hydroxyethyl)amino]-1,5-dimethoxy-benzol, 2,3-Diamino-6-methoxy-pyridin, 3-Amino-6-methoxy-2-(methyl-amino)-pyridin, 2,6-Diamino-3,5-dimethoxy-pyridin, 3,5-Diamino-2,6-dimethoxy-pyridin, 1,3-Diamino-benzol, 2,4-Diamino-1-(2-hydroxyethoxy)-benzol, 1,3-Diamino-4-(2,3-dihydroxypropoxy)-benzol, 1,3-Diamino-4-(3-hydroxypropoxy)-benzol, 1,3-Diamino-4-(2-methoxyethoxy)-benzol, 2,4-Diamino-1,5-di(2-hydroxyethoxy)-benzol, 1-(2-Aminoethoxy)-2,4-diamino-benzol, 2-Amino-1-(2-hydroxyethoxy)-4-methylamino-benzol, 2,4-Diaminophenoxyessigsäure, 3-[Di(2-hydroxyethyl)amino]-anilin, 4-Amino-2-di[(2-hydroxyethyl)amino]-1-ethoxy-benzol, 5-Methyl-2-(1-methylethyl)-phenol, 3-[(2-Hydroxyethyl)amino]-anilin, 3-[(2-Aminoethyl)-amino]-anilin, 1,3-Di(2,4-diaminophenoxy)-propan, Di(2,4-diamino-phenoxy)-methan, 1,3-Diamino-2,4-dimethoxy-benzol, 2,6-Bis(2-hydroxyethyl)amino-toluol, 4-Hydroxyindol, 3-Dimethylamino-phenol, 3-Diethylamino-phenol, 5-Amino-2-methyl-phenol, 5-Amino-4-fluor-2-methyl-phenol, 5-Amino-4-methoxy-2-methyl-phenol, 5-Amino-4-ethoxy-2-methyl-phenol, 3-Amino-2,4-dichlor-phenol, 5-Amino-2,4-dichlor-phenol, 3-Amino-2-methyl-phenol, 3-Amino-2-chlor-6-methyl-phenol, 3-Amino-phenol, 2-[(3-Hydroxyphenyl)-amino]-acetamid, 5-[(2-Hydroxyethyl)amino]-4-methoxy-2-methyl-phenol, 5-[(2-Hydroxyethyl)amino]-2-methyl-phenol, 3-[(2-Hydroxyethyl)amino]-phenol, 3-[(2-Methoxyethyl)amino]-phenol, 5-Amino-2-ethyl-phenol, 5-Amino-2-methoxy-phenol, 2-(4-Amino-2-hydroxyphenoxy)-ethanol, 5-[(3-Hydroxypropyl)amino]-2-methyl-phenol, 3-[(2,3-Dihydroxypropyl)-amino]-2-methyl-phenol, 3-[(2-Hydroxyethyl)-amino]-2-methyl-phenol, 2-Amino-3-hydroxy-pyridin, 2,6-Dihydroxy-3,4-dimethylpyridin, 5-Amino-4-chlor-2-methyl-phenol, 1-Naphthol, 2-Methyl-1-naphthol, 1,5-Dihydroxy-naphthalin, 1,7-Dihydroxy-naphthalin, 2,3-Dihydroxy-naphthalin, 2,7-Dihydroxy-naphthalin, 2-Methyl-1-naphthol-acetat, 1,3-Dihydroxybenzol, 1-Chlor-2,4-dihydroxy-benzol, 2-Chlor-1,3-dihydroxy-benzol, 1,2-Dichlor-3,5-dihydroxy-4-methyl-benzol, 1,5-Dichlor-2,4-dihydroxybenzol, 1,3-Dihydroxy-2-methyl-benzol, 3,4-Methylendioxy-phenol, 3,4-Methylendioxy-anilin, 5-[(2-Hydroxyethyl)amino]-1,3-benzo-dioxol, 6-Brom-1-hydroxy-3,4-methylendioxy-benzol, 3,4-Diamino-benzoesäure, 3,4-Dihydro-6-hydroxy-1,4(2H)-benzoxazin, 6-Amino-3,4-dihydro-1,4(2H)-benzoxazin, 3-Methyl-1-phenyl-5-pyrazolon, 5,6-Dihydroxy-indol, 5,6-Dihydroxy-indolin, 5-Hydroxy-indol, 6-Hydroxy-indol, 7-Hydroxy-indol und 2,3-Indolindion in Betracht.

Als Persulfatsalze kommen zum Beispiel Kaliumpersulfat, Natriumpersulfat oder Ammoniumpersulfat sowie deren Mischungen in Betracht.

Die Persulfatsalze sind in dem gebrauchsfertigen Färbemittel (A) in einer Gesamtmenge von etwa 0,01 bis 10 Gewichtsprozent, vorzugsweise etwa 0,1 bis 5 Gewichtsprozent, enthalten.

Weiterhin kann das erfindungsgemäße Färbemittel zusätzlich zu den Verbindungen der Formel (I) sowie den Kupplersubstanzen gegebenenfalls zusätzlich weitere übliche, physiologisch unbedenkliche, direktziehende Farbstoffe aus der Gruppe der kationischen und anionischen Farbstoffe, der Dispersionsfarbstoffe, der Azofarbstoffe, der Chinonfarbstoffe und der Triphenylmethanfarbstoffe enthalten.

Die direktziehenden Farbstoffe sind in dem gebrauchsfertigen Färbemittel (A) in einer Menge von etwa 0,01 bis 10 Gewichtsprozent, vorzugsweise etwa 0,1 bis 5 Gewichtsprozent, enthalten.

Weiterhin kann das erfindungsgemäße Färbemittel zusätzlich zu den Verbindungen der Formel (I) gegebenenfalls weitere übliche Entwicklersubstanzen enthalten, wie zum Beispiel 1,4-Diamino-benzol (p-Phenylendiamin), 1,4-Diamino-2-methyl-benzol (p-Toluylendiamin), 1,4-Diamino-2-(thiophen-2-yl)benzol, 1,4-Diamino-2-(thiophen-3-yl)benzol, 4-(2,5-Diaminophenyl)-2-((diethylamino)methyl)-thiophen, 2-Chlor-3-(2,5-diaminophenyl)thiophen, 1,4-Diamino-2-(pyridin-3-yl)benzol, 2,5-Diaminobiphenyl, 2,5-Diamino-4'-(1-methylethyl)-1,1'-biphenyl, 2,3',5-Triamino-1,1'-biphenyl, 1,4-Diamino-2-methoxymethyl-benzol, 1,4-Diamino-2-aminomethyl-benzol, 1,4-Diamino-2-((phenylamino)methyl)benzol, 1,4-Diamino-2-((ethyl-(2-hydroxyethyl)-amino)methyl)benzol, 1,4-Diamino-2-hydroxymethyl-benzol, 4-[Di(2-hydroxyethyl)amino]-anilin, 4-(((4-Aminophenyl)-methyl)amino)anilin, 4-[(4-Amino-phenylamino)-methyl]-phenol, 1,4-Diamino-N-(4-pyrrolidin-1-yl-benzyl)-benzol, 1,4-Diamino-N-furan-3-ylmethyl-benzol, 1,4-Diamino-N-thiophen-2-ylmethyl-benzol, 1,4-Diamino-N-furan-2-ylmethyl-benzol, 1,4-Diamino-N-thiophen-3-ylmethyl-benzol, 1,4-Diamino-N-benzyl-benzol, 1,4-Diamino-2-(1-hydroxyethyl)-benzol, 1,4-Diamino-2-(2-hydroxyethyl)-benzol, 1,3-Bis[(4-aminophenyl)(2-hydroxyethyl)amino]-2-propanol, 1,8-Bis(2,5-diaminophenoxy)-3,6-dioxaoctan, 2,5-Diamino-4'-hydroxy-1,1'-biphenyl, 2,5-Diamino-2'-trifluormethyl-1,1'-biphenyl, 2,4',5-Triamino-1,1'-biphenyl, 4-Amino-phenol, 4-Amino-3-methyl-phenol, 4-Methylamino-phenol, 4-Amino-2-(aminomethyl)-phenol, 4-Amino-2-[(2-hydroxyethyl)-amino]methyl-phenol, 4-Amino-2-(methoxymethyl)-phenol, 5-Amino-salicylsäure, 2,4,5,6-Tetraamino-pyrimidin, 2,5,6-Triamino-4-(1 H)-pyrimidon, 4,5-Diamino-1-(2-hydroxyethyl)-1 H-pyrazol, 4,5-Diamino-1-pentyl-1 H-pyrazol, 4,5-Diamino-1-(phenylmethyl)-1 H-pyrazol, 4,5-Diamino-1-((4-methoxyphenyl)methyl-1 H-pyrazol, 2-Amino-phenol, 2-Amino-6-methyl-phenol, 2-Amino-5-methyl-phenol, 1,2,4-Trihydroxy-benzol, 2,4-Diaminophenol, 1,4-Di-hydroxybenzol oder 2-(((4-Aminophenyl)amino)methyl)-1,4-diamino-benzol.

Die Verbindungen der Formel (I) sowie die Kupplersubstanzen und die zusätzlichen Entwicklersubstanzen sind in dem gebrauchsfertigen Färbemittel (A) jeweils in einer Gesamtmenge von etwa 0,01 bis 10 Gewichtsprozent, vorzugsweise etwa 0,1 bis 5 Gewichtsprozent, enthalten.

Die Verbindungen der Formel (I) und die Kupplersubstanzen werden in der Regel getrennt voneinander aufbewahrt und erst kurz vor der Anwendung miteinander vermischt und mit dem Persulfatsalz versetzt. Es ist jedoch auch möglich, sofern die Verbindungen der Formel (I), die Kupplersubstanzen und das Persulfatsalz in fester Form vorliegen, diese gemeinsam abzupacken und das gebrauchsfertige Färbemittel (A) kurz vor der Anwendung durch Vermischen der Verbindungen der Formel (I), der Kupplersubstanzen und des Persulfatsalzes mit Wasser oder einer die übrigen Bestandteile des Mittels enthaltenden flüssigen Zubereitung herzustellen.
Das erfindungsgemäße Färbemittel besteht somit in der Regel aus mehreren Komponenten, welche vor der Anwendung miteinander vermischt werden. Vorzugsweise liegt das Mittel in Form eines 2-Komponenten-Kits, bestehend aus einer Farbträgermasse (A1), welche die Verbindung der Formel (I) enthält, und einer weiteren Farbträgermasse (A2), welche die Kupplersubstanzen und die Persulfatsalze enthält, oder eines 3-Komponenten-Kits, bestehend aus einer Farbträgermasse (A1), welche die Verbindung der Formel (I) enthält, einer weiteren Farbträgermasse (A2), welche die Kupplersubstanzen enthält, und einer die Persulfatsalze enthaltenden 3. Komponente (A3), vor.

Ein weiterer Gegenstand der vorliegenden Erfindung ist ein Mehrkomponenten-Kit, bestehend aus einem Mittel der Komponente (A1) und einem Mittel der Komponente (A2), wobei das Persulfat auch getrennt von der Komponente (A2) als Komponente (A3) abgepackt sein kann, sowie gegebenenfalls einem Mittel zur Einstellung des pH-Wertes (Alkalisierungsmittel oder Säure). Selbstverständlich können auch die Mittel der Komponenten (A1) und (A2) aus mehreren Einzelkomponenten bestehen, welche erst unmittelbar vor der Anwendung miteinander vermischt werden. Ebenfalls ist ein 2-Komponenten-Kit möglich, dessen 1. Komponente aus einem die Verbindungen der Formel (I), die Kupplersubstanzen und die Persulfatsalze sowie gegebenenfalls weitere übliche pulverförmige kosmetische Zusatzstoffe enthaltenden Pulver besteht (sofern die vorgenannten Bestandteile in fester Form vorliegen), und dessen 2. Komponente Wasser oder eine flüssige kosmetische Zubereitung ist und gegebenenfalls ein Mittel zur Einstellung des pH-Wertes enthält.
Besonders bevorzugt ist jedoch ein 2-Komponenten-Kit, bestehend aus einem Mittel der Komponente (A1) und einem Mittel der Komponente (A2).

Die vorgenannten direktziehenden Farbstoffe können in der Komponente (A2) in einer Gesamtmenge von etwa 0,02 bis 20 Gewichtsprozent, vorzugsweise 0,2 bis 10 Gewichtsprozent, eingesetzt werden, während die zusätzlichen Entwicklersubstanzen und die Kupplersubstanzen in der jeweiligen Farbträgermasse (Komponente (A1) bzw. Komponente (A2)) jeweils in einer Gesamtmenge von etwa 0,02 bis 20 Gewichtsprozent, vorzugsweise etwa 0,2 bis 10 Gewichtsprozent, enthalten sein können.

Die Zubereitungsform für die Komponenten (A1) und (A2) sowie das gebrauchsfertige Färbemittel (A) kann beispielsweise eine Lösung, insbesondere eine wässrige oder wässrig-alkoholische Lösung, eine Creme, ein Gel oder eine Emulsion sein. Ihre Zusammensetzung stellt eine Mischung der Verbindung der Formel (I) beziehungsweise der Kupplersubstanzen mit den für solche Zubereitungen üblichen Zusätzen dar.

Übliche in Färbemitteln verwendete Zusätze in Lösungen, Cremes, Emulsionen, Gelen oder Aerosolschäumen sind zum Beispiel Lösungsmittel wie Wasser, niedere aliphatische Alkohole, beispielsweise Ethanol, n-Propanol und Isopropanol oder Glykole wie Glycerin und 1,2-Propandiol, weiterhin Netzmittel oder Emulgatoren aus den Klassen der anionischen, kationischen, amphoteren oder nichtionogenen oberflächenaktiven Substanzen wie Fettalkoholsulfate, oxethylierte Fettalkoholsulfate, Alkylsulfonate, Alkylbenzolsulfonate, Alkyltrimethylammoniumsalze, Alkylbetaine, oxethylierte Fettalkohle, oxethylierte Nonylphenole, Fettsäurealkanolamide, oxethylierte Fettsäureester, ferner Verdicker wie höhere Fettalkohole, Stärke oder Cellulosederivate, Parfüme, Haarvorbehandlungsmittel, Konditionierer, Haarquellmittel, Konservierungsstoffe, weiterhin Vaseline, Paraffinöl und Fettsäuren sowie außerdem Pflegestoffe wie kationische Harze, Lanolinderivate, Cholesterin, Pantothensäure und Betain. Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent (jeweils bezogen auf die Komponente (A1) bzw. (A2)), die Verdicker in einer Menge von etwa 0,1 bis 25 Gewichtsprozent (jeweils bezogen auf die Komponente (A1) bzw. (A2)) und die Pflegestoffe in einer Konzentration von etwa 0,1 bis 5,0 Gewichtsprozent (jeweils bezogen auf die Komponente (A1) bzw. (A2)).

Der pH-Wert des gebrauchsfertigen Färbemittels (A) sowie der Farbträgermassen (A1) und (A2) beträgt jeweils etwa 3 bis 12, vorzugsweise etwa 3 bis 10, wobei sich der pH-Wert des gebrauchsfertigen Färbemittels (A) in der Regel bei der Mischung der Einzelkomponenten (zum Beispiel der Komponente (A1) mit der Komponente (A2)) einstellt. Der pH-Wert des gebrauchsfertigen Färbemittels (A) sowie der Farbträgermassen (A1) und (A2) beträgt vorzugsweise etwa 3 bis 7 wenn als Kupplersubstanzen Diaminobenzol-Derivate verwendet werden, und etwa 6 bis 10 wenn als Kupplersubstazen Aminophenol- oder Dihydroxybenzol-Derivate verwendet werden.

Zur Einstellung des für die Färbung gewünschten pH-Wertes der Komponenten (A1) und (A2) sowie des gebrauchsfertigen Färbemittels (A) können -falls erforderlich- jedoch auch alkalisierende Mittel, wie zum Beispiel Ammoniak, Alkalihydroxide, Erdalkalihydroxide, Alkaliacetate, Erdalkaliacetate, Alkalicarbonate oder Erdalkalicarbonate, oder Säuren, wie zum Beispiel Milchsäure, Essigsäure, Weinsäure, Phosphorsäure, Salzsäure, Zitronensäure, Ascorbinsäure oder Borsäure, zugesetzt werden.

Das gebrauchsfertige Färbemittel wird unmittelbar vor der Anwendung durch Vermischen der Komponenten (A1) und (A2) bzw. (A1) und (A2) und (A3) -gegebenenfalls unter Zusatz eines Alkalisierungsmittel oder einer Säure- hergestellt und sodann auf die Faser, insbesondere menschliche Haare, aufgetragen. Je nach gewünschter Farbtiefe läßt man diese Mischung etwa 5 bis 60 Minuten, vorzugsweise etwa 15 bis 30 Minuten, bei einer Temperatur von etwa 20 bis 50 °C, insbesondere bei etwa 30 bis 40 °C einwirken. Anschließend wird die Faser mit Wasser gespült, gegebenenfalls mit einem Shampoo gewaschen und sodann getrocknet.

Das erfindungsgemäße Färbemittel ermöglicht eine gleichmäßige, besonders brillante, intensive, und dauerhafte Färbung der Fasern, insbesondere von Keratinfasern, wie zum Beispiel menschlichen Haaren, wobei eine breite Farbpalette von gelben bis blauen Farbtönen möglich ist. Die Beständigkeit gegenüber Schweiss wird in besonders hohe Masse erfüllt.

Die nachfolgenden Beispiele sollen den Gegenstand näher erläutern, ohne ihn auf diese Beispiele zu beschränken.

### Beispiele

### Beispiel 1a: Synthese von 3,4-Dimethyl-2(3H)-thiazolon-hydrazon-Hydrochlorid

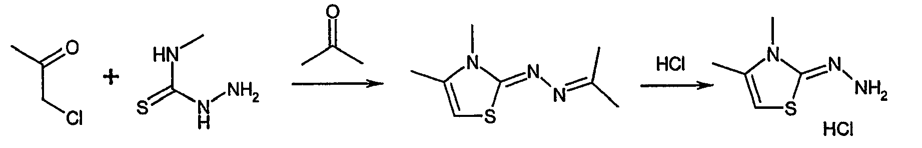

### Stufe A: 3,4-Dimethyl-2(3H)-thiazolon-(1-methylethyliden)hydrazon

21 g (200 mmol) 4-Methyl-3-thiosemicarbazid werden in 1000 ml Aceton 2 Stunden lang unter Rückfluss gekocht. Dann wird die Lösung tropfenweise mit 20,4 g (220 mmol) Chloraceton versetzt. Die Reaktionsmischung wird sodann 7 Stunden lang unter Rückfluss gekocht, und anschließend eingeengt. Das so erhaltene Rohprodukt wird aus Aceton umkristallisiert. Es werden 23 g eines orangen Pulvers (63% der Theorie) erhalten.
Schmelzpunkt: 139 -139,6 °C
¹H-NMR (DMSO, 300 MHz): δ = 6,72 (s, breit, 1H, H-C(5)); δ = 3,67 (s, 3H, N-CH₃); δ = 2,27 (d, J=0,9 Hz, 3H, CH₃-C(4)); δ = 2,17 (s, 3H, CH₃); δ = 2,07 (s, 3H, CH₃).
¹³C-NMR (DMSO, 300 MHz): 169,16; 164,14; 139,02 (C(4)); 103,36 (C(5)); 34,47 (CH₃N); 24,60; 19,91; 13,53 (CH₃ (C4)).
MS (ESI): 184 (M⁺ +1)

### Stufe B: 3,4-Dimethyl-2(3H)-thiazolon-hydrazon Hydrochlorid

3,5 g (19 mmol) 3,4-Dimethyl-2(3H)-thiazolon-(1-methylethyliden) hydrazon aus Stufe A werden in 60 ml 6M Salzsäure bei 50°C 30 Minuten lang erwärmt. Die Reaktionsmischung wird anschließend eingeengt und das Rohprodukt wird sodann aus Ethanol umkristallisiert. Es werden 2 g (60% der Theorie) eines rosa Pulvers erhalten.
Schmelzpunkt: 156,4 - 156,6 °C
¹H-NMR (DMSO, 300 MHz): δ = 6,58 (q, J=0,9 Hz, 1 H, H-C(5)); δ = 3,41 (s, 3H, N-CH₃); δ = 2,18 (d, J=0,9Hz, 3H, CH₃-C(4)).
MS (ESI): 144 (M⁺+1).
¹³C-NMR (DMSO, 300 MHz): 172,30 (C(2)); 138,79 (C(4)); 101,43 (C(5)); 32,92 (CH₃N); 13,40 (CH₃ (C4)).
CHN-Analyse:
(C₅H₉N₃S (0,96 HCl) (0,5 EtOH)):

| | %C | % H | % N | %S | %Cl |
|---|---|---|---|---|---|
| berechnet: | 35,81 | 6,49 | 20,88 | 15,93 | 16,90 |
| gefunden: | 35,20 | 6,300 | 21,00 | 15,4 | 16,80 |

### Beispiele 1b-1g: Synthese von 2(3H)-Thiazolon-hydrazonen der Formel (I) (allgemeine Synthese Vorschrift)

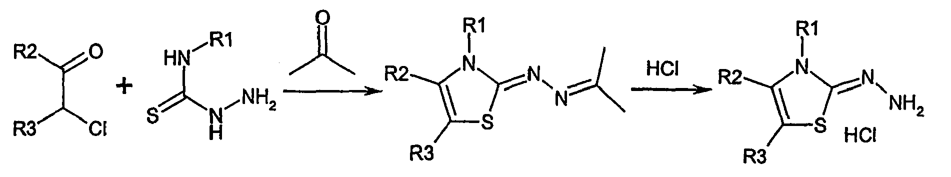

**Stufe A:** 4 mmol substituiertes Thiosemicarbazid werden in 20 ml Aceton 2 Stunden lang unter Rückfluss gekocht. Dann wird die Lösung tropfenweise mit 4,4 mmol α-Chlor-keton versetzt. Die Reaktionsmischung wird sodann 7 Stunden lang unter Rückfluss gekocht, und anschließend eingeengt. Das so erhaltene 2(3H)-Thiazolon-1-(methylethyliden)-hydrazon Derivat wird aus Aceton umkristallisiert.
**Stufe B:** 2 mmol des 2(3H)-Thiazolon-1-(methylethyliden)-hydrazon Derivates aus Stufe A werden in 10 ml 6M Salzsäure bei 50 °C 30 Minuten lang erwärmt. Die Reaktionsmischung wird anschließend eingeengt und das Rohprodukt wird sodann aus Ethanol oder Butanol umkristallisiert.

### 1b.) 3-Methyl-4-phenyl-2(3H)-thiazolon-hydrazon Hydrochlorid

Aus 4-Methyl-3-thiosemicarbazid und Phenacylchlorid
¹H-NMR (DMSO/D₂O, 300 MHz): δ = 7,49-7,42 (m, 5H, phenyl); δ = 6,84 (s, 1H, H-C(5)); δ = 3,31 (s, 3H, N-CH₃).
ESI-MS:205 [M]⁺(100)

### 1 c.) 4-tert-Butyl-3-methyl-2(3H)-thiazolon-hydrazon Hydrochlorid

Aus 4-Methyl-3-thiosemicarbazid und 1-Chlor-3,3-dimethyl-2-butanon ¹H-NMR (DMSO/D₂O, 300 MHz): δ = 6,55 (s, 1H, H-C(5)); 8 = 3,60 (s, 3H N-CH₃); δ = 1,31 (s,9H, (CH₃)₃).
ESI-MS: 185 [M]⁺ (100)

### 1d.) 4-Methyl-3-(2-propenyl)-2(3H)-thiazolon-hydrazon Hydrochlorid

Aus 4-(2-propenyl)-3-thiosemicarbazid und Chloraceton
¹H-NMR (DMSO/D₂O, 300 MHz): δ = 6,58 (s, 1H,H-C(5)); δ = 5,94-5,81 (m, 1H, Allyl); δ = 5,22 (dd, 1 H, J=0,9 Hz, J=10,5 Hz, Allyl); δ = 4,94 (dd, 1 H, J=0,9 Hz, J=17,1 Hz, Allyl); δ = 4,57 (m, 2H, N-CH₂); δ = 2,16 (s, 3H, CH₃-C(4)).
ESI-MS: 169 [M]⁺ (100)

### 1e.) 4-Phenyl-3-(2-propenyl)-2(3H)-thiazolon-hydrazon Hydrochlorid

Aus 4-(2-propenyl)-3-thiosemicarbazid und Phenacylchlorid
¹H-NMR (DMSO/D₂O, 300 MHz): δ = 7,50-7,42 (m, 5H, phenyl); δ = 6,81 (s, 1 H, H-C(5)); δ = 5,77-5,63 (m, 1 H, Allyl); δ = 5,15 (dd, 1 H, J=0,9 Hz, J=10,5 Hz, Allyl); δ = 4,80 (dd, 1 H, J=0,9 Hz, J=17,1 Hz, Allyl); δ = 4,40 (m, 2H, N-CH₂); δ = 1,27 (s, 9H, CH₃-C(4)).
ESI-MS: 231 [M]⁺ (100)

### 1f.) 4-tert-Butyl-3-(2-propenyl)-2(3H)-thiazolon-hydrazon Hydrochlorid

Aus 4-(2-propenyl)-3-thiosemicarbazid und 1-Chlor-3,3-dimethyl-2-butanon
¹H-NMR (DMSO/D₂O, 300 MHz): δ = 6,55 (s, 1H, H-C(5)); δ = 5,90-5,77 (m, 1 H, Allyl); δ = 5,21 (d, 1 H, J=9,0 Hz, Allyl); δ = 4,81-4,75 (m, 3H, Allyl); δ = 1,31 (s, 9H, (CH₃)₃).
ESI-MS: 211 [M]⁺ (100)

### 1g.) 3,4,5-Trimethyl-2(3H)-thiazolon-hydrazon Hydrochlorid

Aus 4-Methyl-3-thiosemicarbazid und 3-Chlor-2-butanon
¹H-NMR (DMSO/D₂O, 300 MHz): δ = 3,55 (s, 3H, N-CH₃); δ = 2,16 (s, 3H, CH₃; δ = 2,12 (s, 3H, CH₃).
ESI-MS: 157 [M]⁺ (100)

### Beispiele 2 - 26: Färbemittel mit 3-Methyl-2(3H)-benzothiazolon-hydrazon Hydrochlorid

### Komponente (A1)

| | |
|---|---|
| 4,00 g | Decylpolyglucose (Plantaren® 2000), 50 %ige wässrige Lösung |
| 0,20 g | Ethylendiaminotetraessigsäure-Dinatriumsalz-Hydrat |
| 5,00 g | Ethanol |
| 0,58 g | 3-Methyl-2(3H)-benzothiazolon-hydrazon-Hydrochlorid Hydrat |
| ad 100,00 g | Wasser, entmineralisiert |

### Komponente (A2)

| | |
|---|---|
| Y g | Kupplersubstanz gemäß Tabelle 1 |
| 0,40 g | Kaliumpersulfat |

Bei Raumtemperatur (20-25 °C) oder unter leichtem Erwärmen (35-40 °C) werden die vorstehend genannten Bestandteile homogen miteinander vermischt. Der pH-Wert des gebrauchsfertigen Färbemittels (A) wird -falls erforderlich- mit Natronlauge, Natriumcarbonat, Ammoniak oder Zitronensäure auf den in der Tabelle 1 angegebenen Wert eingestellt.

Das gebrauchsfertige Haarfärbemittel wird auf gebleichtes Haar aufgetragen und mit einem Pinsel gleichmäßig verteilt. Nach einer Einwirkungszeit von 30 Minuten bei 40 °C wird das Haar mit lauwarmem Wasser gespült, mit einem Shampoo gewaschen, mit lauwarmem Wasser gespült und sodann getrocknet.

Die Einsatzmenge der Kupplersubstanz sowie die erhaltenen Färbungen sind in der nachfolgenden Tabelle 1 zusammengefaßt.

**Tabelle 1:**

| **Bsp. Nr.** | **Verwendete Kupplersubstanz (Menge in g)** | | **pH-Wert** | **Farbton** |
|---|---|---|---|---|
| **2** | 1,3-Diamino-benzol | (0,27 g) | 7,0 | rubinrot |
| **3** | 2,4-Diamino-1-(2-hydroxyethoxy)-benzol-sulfat | (0,66 g) | 7,5 | mahagoni-farben |
| **4** | 2-Amino-4-[(2-hydroxyethyl)-amino]-anisol sulfat | (0,74 g) | 6,0 | mahagoni-farben |
| **5** | 1,3-Di(2,4-diaminophenoxy)-propan-tetrahydrochlorid | (1,08 g) | 6,2 | mahagoni-farben |
| **6** | 1,3-Diamino-4-methoxybenzol | (0,34 g) | 7,7 | mahagoni-farben |
| **7** | 1,3-Diamino-4-methylbenzol | (3,0 g) | 6,1 | rubinrot |
| **8** | N-(3-Dimethylamino-phenyl)-harnstoff | (0,44 g) | 6,6 | violett |
| **9** | 3-Aminophenol | (0,27 g) | 3,6 | rubinrot |
| **10** | 5-Amino-2-methyl-phenol | (0,31 g) | 3,9 | kupferfarben |
| **11** | 3-Dimethylamino-phenol | (0,34 g) | 3,6 | himberrot |
| **12** | 1,3-Dihydroxybenzol | (0,27 g) | 3,8 | kupferfarben |
| **13** | 1-Naphthol | (0,36 g) | 3,7 | orange |
| **14** | 1,5-Dihydroxy-naphthalin | (0,40 g) | 3,2 | kupferfarben |
| **15** | 1,7-Dihydroxy-naphthalin | (0,40 g) | 3,7 | rosarot |
| **16** | 8-Hydroxychinolin | (0,36 g) | 3,4 | kupferfarben |
| **17** | 2,3-Diaminopyridin | (0,27 g) | 9,6 | kirschrot |
| **18** | 2-Amino-3-hydroxypyridin | (0,27 g) | 9,1 | rot-violett |
| **19** | 4-Amino-1H-indol | (0,33 g) | 7,5 | violett |
| **20** | 1,2-Diaminobenzol | (0,27 g) | 6,1 | braun |
| **21** | 2-Amino-phenol | (0,27 g) | 3,6 | braun-mahagoni |
| **22** | 1,3-Diamino-benzol | (0,14 g) | 6,5 | rubinrot |
| | 3-Aminophenol | (0,14 g) | | |
| **23** | 1,3-Diamino-benzol | (0,14 g) | 6,2 | rubinrot |
| | 1,3-Dihydroxybenzol | (0,14 g) | | |
| **24** | 1,3-Diamino-benzol | (0,14 g) | 7,7 | mahagoni-farben |
| | N-(3-Dimethylamino-phenyl)-harnstoff | (0,22 g) | | |
| **25** | N-(3-Dimethylamino-phenyl)-harnstoff | (0,22 g) | 6,4 | violett |
| | 1,3-Dihydroxybenzol | (0,14 g) | | |
| **26** | 1,3-Diamino-benzol | (0,05 g) | 9,6 | mahagoni-farben |
| | 2,4-Diamino-1-(2-hydroxyethoxy)-benzol-sulfat | (0,12 g) | | |
| | 5-Amino-2-methyl-phenol | (0,06 g) | | |
| | 3-Aminophenol | (0,05 g) | | |
| | 1,3-Dihydroxybenzol | (0,05 g) | | |

### Beispiele 27-35: Färbemittel mit 3,4-Dimethyl-2(3H)-thiazolon-hydrazon Hydrochlorid

### Komponente (A1)

| | |
|---|---|
| 4,00 g | Decylpolyglucose (Plantaren® 2000), 50 %ige wässrige Lösung |
| 0,20 g | Ethylendiaminotetraessigsäure-Dinatriumsalz-Hydrat |
| 5,00 g | Ethanol |
| 0,45 g | 3,4-Dimethyl-2(3H)- thiazolon-hydrazon Hydrochlorid |
| ad 100,00 g | Wasser, entmineralisiert |

### Komponente (A2)

| | |
|---|---|
| Y g | Kupplersubstanz gemäß Tabelle 2 |
| 0,40 g | Kaliumpersulfat |

Bei Raumtemperatur (20-25 °C) oder unter leichtem Erwärmen (35-40 °C) werden die vorstehend genannten Bestandteile homogen miteinander vermischt. Der pH-Wert des gebrauchsfertigen Färbemittels (A) wird -falls erforderlich- mit Natronlauge, Natriumcarbonat, Ammoniak oder Zitronensäure auf den in der Tabelle 2 angegebenen Wert eingestellt.

Das gebrauchsfertige Haarfärbemittel wird auf gebleichtes Haar aufgetragen und mit einem Pinsel gleichmäßig verteilt. Nach einer Einwirkungszeit von 30 Minuten bei 40 °C wird das Haar mit lauwarmem Wasser gespült, mit einem Shampoo gewaschen, mit lauwarmem Wasser gespült und sodann getrocknet.

Die Einsatzmenge der Kupplersubstanz sowie die erhaltenen Färbungen sind in der nachfolgenden Tabelle 2 zusammengefaßt.

**Tabelle 2:**

| **Bsp. Nr.** | **Verwendete Kupplersubstanz (Menge in g)** | | **pH-Wert M** | **Farbton** |
|---|---|---|---|---|
| **27** | 1,3-Diamino-benzol | (0,27 g) | 7,2 | bordeauxrot |
| **28** | 2,4-Diamino-1-(2-hydroxyethoxy)-benzolsulfat | (0,67 g) | 6,7 | dunkel bordeauxrot |
| **29** | N-(3-Dimethylamino-phenyl)-harnstoff | (0,44 g) | 7,3 | blau |
| **30** | 3-Aminophenol | (0,27 g) | 7,2 | himberrot |
| **31** | 5-Amino-2-methyl-phenol | (0,31 g) | 6,6 | rubin-rot |
| **32** | 5-((2-hydroxyethyl)amino)-2-methyl-Phenol-sulfat(2:1) | (0,54 g) | 6,8 | orange |
| **33** | 1,3-Dihydroxybenzol | (0,27 g) | 9,9 | rosa-Kupfer |
| **34** | 1-Naphthol | (0,36 g) | 9,7 | rosa |
| **35** | 1,3-Diamino-benzol | (0,05 g) | 9,8 | dunkel violett- |
| | 2,4-Diamino-1-(2-hydroxyethoxy)-benzol sulfat | (0,12 g) | | Aubergine |
| | 5-Amino-2-methyl-phenol | (0,06 g) | | |
| | 3-Aminophenol | (0,05 g) | | |
| | 1,3-Dihydroxybenzol | (0,05 g) | | |

### Beispiele 36-65: Färbemittel mit 2(3H)-Thiazolon-hydrazon der Formel (I) (Beisp. 1b-1g)

### Komponente (A1)

| | |
|---|---|
| 4,00 g | Decylpolyglucose (Plantaren® 2000), 50 %ige wässrige Lösung |
| 0,20 g | Ethylendiaminotetraessigsäure-Dinatriumsalz-Hydrat |
| 5,00 g | Ethanol |
| X g | 2(3H)-Thiazolon-hydrazon der Formel (I) (1 b-1g) |
| ad 100,00 g | Wasser, entmineralisiert |

### Komponente (A2)

| | |
|---|---|
| Y g | Kupplersubstanz gemäß Tabelle 3 |
| 0,80 g | Kaliumpersulfat |

Bei Raumtemperatur (20-25 °C) oder unter leichtem Erwärmen (35-40 °C) werden die vorstehend genannten Bestandteile homogen miteinander vermischt. Der pH-Wert des gebrauchsfertigen Färbemittels (A) wird -falls erforderlich- mit Natronlauge, Natriumcarbonat, Ammoniak oder Zitronensäure auf den in der Tabelle 3 angegebenen Wert eingestellt.

Das gebrauchsfertige Haarfärbemittel wird auf gebleichtes Büffelhaar aufgetragen und mit einem Pinsel gleichmäßig verteilt. Nach einer Einwirkungszeit von 30 Minuten bei 40 °C wird das Haar mit lauwarmem Wasser gespült, mit einem Shampoo gewaschen, mit lauwarmem Wasser gespült und sodann getrocknet.

Die Einsatzmenge der 2(3H)-Thiazolon-hydrazon der Formel (I) (1 b-1 g) und der Kupplersubstanz sowie die erhaltenen Färbungen sind in der nachfolgenden Tabelle 3 zusammengefaßt.

**Tabelle 3:**

| Bsp. Nr. | Derivat der Formel (I) gemäss Beispiel Nr. (Menge in g) | Verwendete Kupplersubstanz (Menge in g) | pH-Wert | Farbton |
|---|---|---|---|---|
| 36 | Beispiel 1 b (0,60 g) | 1,3-Diamino-benzol (0,27 g) | 9,6 | bordeauxrot |
| 37 | Beispiel 1 c (0,55 g) | 1,3-Diamino-benzol (0,27 g) | 9,6 | bordeauxrot |
| 38 | Beispiel 1 d (0,51 g) | 1,3-Diamino-benzol (0,27 g) | 9,6 | bordeauxrot |
| 39 | Beispiel 1 e (0,67 g) | 1,3-Diamino-benzol (0,27 g) | 9,6 | bordeauxrot |
| 40 | Beispiel 1f (0,62 g) | 1,3-Diamino-benzol (0,27 g) | 9,6 | bordeauxrot |
| 41 | Beispiel 1 g (0,48 g) | 1,3-Diamino-benzol (0,27 g) | 9,2 | violett-Aubergine |
| 42 | Beispiel 1b (0,60 g) | 2,4-Diamino-1-(2-hydroxy-ethoxy)-benzol sulfat (0,67 g) | 9,6 | bordeauxrot |
| 43 | Beispiel 1c (0,55 g) | 2,4-Diamino-1-(2-hydroxy-ethoxy)-benzol sulfat (0,67 g) | 9,6 | bordeauxrot |
| 44 | Beispiel 1d (0,51 g) | 2,4-Diamino-1-(2-hydroxy-ethoxy)-benzol sulfat (0,67 g) | 9,6 | bordeauxrot |
| 45 | Beispiel 1e (0,67 g) | 2,4-Diamino-1-(2-hydroxy-ethoxy)-benzol sulfat (0,67 g) | 9,6 | bordeauxrot |
| 46 | Beispiel 1f (0,62 g) | 2,4-Diamino-1-(2-hydroxy-ethoxy)-benzol sulfat (0,67 g) | 9,6 | bordeauxrot |
| 47 | Beispiel 1g (0,48 g) | 2,4-Diamino-1-(2-hydroxy-ethoxy)-benzol sulfat (0,67 g) | 9,4 | violett-Aubergine |
| 48 | Beispiel 1b (0,60 g) | N-(3-Dimethylaminophenyl)-harnstoff (0,44 g) | 9,5 | blau |
| 49 | Beispiel 1c (0,55 g) | N-(3-Dimethylaminophenyl)-harnstoff (0,44 g) | 9,5 | blau |
| 50 | Beispiel 1d (0,51 g) | N-(3-Dimethylaminophenyl)-harnstoff (0,44 g) | 9,5 | blau |
| 51 | Beispiel 1e (0,67 g) | N-(3-Dimethylaminophenyl)-harnstoff (0,44 g) | 9,5 | blau |
| 52 | Beispiel 1f (0,62 g) | N-(3-Dimethylaminophenyl)-harnstoff (0,44 g) | 9,5 | blau |
| 53 | Beispiel 1g (0,48 g) | N-(3-Dimethylaminophenyl)-harnstoff (0,44 g) | 9,4 | blau |
| 54 | Beispiel 1b (0,60 g) | 3-Aminophenol (0,27 g) | 9,6 | himberrot |
| 55 | Beispiel 1c (0,55 g) | 3-Aminophenol (0,27 g) | 9,6 | himberrot |
| 56 | Beispiel 1d (0,51 g) | 3-Aminophenol (0,27 g) | 9,6 | himberrot |
| 57 | Beispiel 1e (0,67 g) | 3-Aminophenol (0,27 g) | 9,6 | himberrot |
| 58 | Beispiel 1f (0,62 g) | 3-Aminophenol (0,27 g) | 9,6 | himberrot |
| 59 | Beispiel 1g (0,48 g) | 3-Aminophenol (0,27 g) | 9,3 | violett-rot |
| 60 | Beispiel 1b (0,60 g) | 1,3-Dihydroxybenzol (0,27 g) | 9,6 | rot-orange |
| **61** | Beispiel 1c (0,55 g) | 1,3-Dihydroxybenzol (0,27 g) | 9,6 | rot-orange |
| 62 | Beispiel 1d (0,51 g) | 1,3-Dihydroxybenzol (0,27 g) | 9,6 | rot-orange |
| 63 | Beispiel 1e (0,67 g) | 1,3-Dihydroxybenzol (0,27 g) | 9,6 | rot-orange |
| 64 | Beispiel 1f (0,62 g) | 1,3-Dihydroxybenzol (0,27 g) | 9,6 | rot-orange |
| 65 | Beispiel 1g (0,48 g) | 1,3-Dihydroxybenzol (0,27 g) | 9,6 | himberrot |

Alle in der vorliegenden Anmedlung angegebenen Prozentzahlen stellen -soweit nicht anders angegeben- Gewichtsprozente dar.

## Patentansprüche

1. Mittel zur Färbung von keratinfasern (A), **dadurch gekennzeichnet, dass** es (a) mindestens ein Hydrazon-Derivat der Formel (I) oder dessen physiologisch verträgliches Salz, 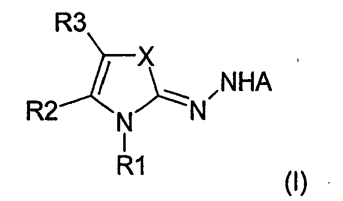 worin X gleich Sauerstoff oder Schwefel ist;
**A** Wasserstoff, eine Acetylgruppe, eine Trifluoracetylgruppe, eine Formylgruppe, eine (C₁-C₆)-Alkylsulfonylgruppe oder eine Arylsulfonylgruppe darstellt;
**R1** gleich einer gesättigten oder ungesättigten (C₁-C₁₂)-Alkylgruppe, einer mit einem Halogenatom (F, Cl, Br, J) substituierten (C₁-C₁₂)-Alkylgruppe, einer Hydroxy-(C₁-C₁₂)-alkylgruppe, einer Amino-(C₁-C₁₂)-alkylgruppe, einer Sulfonsäure-(C₁-C₁₂)-alkyl-gruppe, einer Formylgruppe, einer C(O)-(C₁-C₁₂)-Alkylgruppe, einer C(O)-Phenylgruppe, einer C(O)NH-(C₁₋C₁₂)-Alkylgruppe, einer C(O)NH-Phenylgruppe, einer substituierten oder unsubstituierten Phenylgruppe, oder einer Benzylgruppe ist;
**R2** und **R3** gleich oder verschieden sein können und unabhängig voneinander Wasserstoff, ein Halogenatom (F, Cl, Br, J), eine gesättigte oder ungesättigte (C₁-C₁₂)-Alkylgruppe, eine mit einem Halogenatom (F, Cl, Br, J) substituierte (C₁-C₁₂)-Alkylgruppe, eine Hydroxy-(C₁-C₁₂)-alkylgruppe, eine (C₁-C₁₂)-Alkoxygruppe, eine Cyanogruppe, eine Nitrogruppe, eine Aminogruppe, eine (C₁-C₁₂)-Alkylaminogruppe, eine (C₁-C₁₂)-Dialkylaminogruppe, eine Carbonsäure, eine C(O)O-(C₁-C₁₂)-Alkylgruppe, eine substituierte oder unsubstituierte C(O)O- Phenylgruppe, eine substituierte oder unsubstituierte Phenylgruppe oder eine Naphthylgruppe darstellen, oder **R2** und **R3** gemeinsam mit dem Restmolekül ein heterozyklisches oder carbozyklisches, gesättigtes oder ungesättigtes, substituiertes oder unsubstituiertes Ringsystem bilden;
(b) mindestens eine an sich bekannten Kupplersubstanz oder deren physiologisch verträgliches Salz,
sowie (c) als oxidatives Mittel ein Persulfatsalz enthält.

2. Mittel nach Anspruch 1, **dadurch gekennzeichnet, dass X** gleich Schwefel ist, **A** gleich Wasserstoff ist, **R1** gleich einer gesättigten oder ungesättigten (C₁-C₁₂)-Alkylgruppe, einer Hydroxy-(C₁-C₁₂)-alkylgruppe, einer Amino-(C₁-C₁₂)-alkylgruppe, oder einer substituierten oder unsubstituierten Phenylgruppe ist, und **R2** und **R3** unabhängig voneinander Wasserstoff, eine gesättigte oder ungesättigte (C₁-C₁₂)-Alkylgruppe, eine Cyanogruppe, eine Nitrogruppe, eine Aminogruppe, eine (C₁-C₁₂)-Alkylaminogruppe, eine (C₁-C₁₂)-Dialkylaminogruppe, eine C(O)O-Alkylgruppe oder eine substituierte oder unsubstituierte Phenylgruppe oder eine Naphtylgruppe darstellen, oder **R2** und **R3** gemeinsam mit dem Restmolekül ein carbozyklisches, ungesättigtes, substituiertes oder unsubstituiertes Ringsystem, bilden.

3. Mittel nach Anspruch 1 oder 2, **dadurch gekennzeichnet, dass** das Hydrazon-Derivat der Formel (I) ausgewählt ist aus 3-Methyl-2(3H)-thiazolon-hydrazon, 3,4-Dimethyl-2(3H)-thiazolon-hydrazon, 4-tert-Butyl-3-methyl-2(3H)-thiazolon-hydrazon, 3-Methyl-4-phenyl-2(3H)-thiazolon-hydrazon, 3-Methyl-4-(4-tolyl)-2(3H)-thiazolon-hydrazon, 4-(4-Methoxy)-phenyl-3-methyl-2(3H)-thiazolon-hydrazon, 4-(4-Ethoxy)phenyl-3-methyl-2(3H)-thiazolon-hydrazon, 4-(4-Bromphenyl)-3-methyl-2(3H)-thiazolon-hydrazon, 4-(3-Bromphenyl)-3-methyl-2(3H)-thiazolon-hydrazon, 4-(4-Chlorphenyl)-3-methyl-2(3H)-thiazolon-hydrazon, 4-(3-Chlorphenyl)-3-methyl-2(3H)-thiazolon-hydrazon, 3-Methyl-4-(4-nitrophenyl)-2(3H)-thiazolon-hydrazon, 3-Methyl-4-(3-nitrophenyl)-2(3H)-thiazolon-hydrazon, 4-([1,1'-Biphenyl]-4-yl)-3-methyl-2(3H)-thiazolon-hydrazon, 3-Methyl-4-(2-naphthalenyl)-2(3H)-thiazolon-hydrazon, 2-Hydrazono-2,3-dihydro-3-methyl-4-thiazolcarbonsäureethylester, 3,4,5-Trimethyl-2(3H)-thiazolon-hydrazon, 3,4-Dimethyl-5-phenyl-2(3H)-thiazolon-hydrazon, 3,5-Dimethyl-4-phenyl-2(3H)-thiazolon-hydrazon, 3-Methyl-4,5-diphenyl-2(3H)-thiazolon-hydrazon, 5-Ethyl-3-methyl-4-phenyl-2(3H)-thiazolon-hydrazon, 4-(4-Bromphenyl)-3-methyl-5-phenyl-2(3H)-thiazolon-hydrazon, 3-Methyl-5-phenyl-4-(4-tolyl)-2(3H)-thiazolon-hydrazon, 5-(4-Chlorphenyl)-4-phenyl-3-methyl-2(3H)-thiazolon-hydrazon, 5-(4-Chlorphenyl)-4-(4-methoxyphenyl)-3-methyl-2(3H)-thiazolon-hydrazon, 2-Hydrazono-2,3-dihydro-3,4-dimethyl-4-thiazolcarbonsäureethylester, 4-Amino-2-hydrazono-2,3-dihydro-3-methyl-5-thiazolcarbonitril, 3-Ethyl-4,5-dimethyl-2(3H)-thiazolon-hydrazon, 2-Hydrazono-2,3-dihydro-3-ethyl-4-methyl-thiazol-carbonsäureethylester, 5-Methyl-3-(1-methylethyl)-4-phenyl-2(3H)-thiazolon-hydrazon, 4,5-Dimethyl-3-(1-methylethyl)-2(3H)-thiazolon-hydrazon, 3-(1-methylethyl)-4,5-diphenyl-2(3H)-thiazolon-hydrazon, 4,5-Dimethyl-3-propyl-2(3H)-thiazolon-hydrazon, 4,5-Diphenyl-3-propyl-2(3H)-thiazolon-hydrazon, 3-Butyl-4,5-diphenyl-2(3H)-thiazolon-hydrazon, 4,5-Dimethyl-3-(2-methylpropyl)-2(3H)-thiazolon-hydrazon, 3-(2-methylpropyl)-4,5-diphenyl-2(3H)-thiazolon-hydrazon, 3-Hydroxyethyl-2(3H)-thiazolon-hydrazon,3-Hydroxyethyl-4-methyl-2(3H)-thiazolon-hydrazon, 3-Hydroxyethyl-4,5-dimethyl-2(3H)-thiazolon-hydrazon, 3-Aminoethyl-2(3H)-thiazolon-hydrazon, 3-Aminoethyl-4-methyl-2(3H)-thiazolon-hydrazon, 3-Aminoethyl-4,5-dimethyl-2(3H)-thiazolon-hydrazon, 3,4-Diphenyl-2(3H)-thiazolon-hydrazon, 4-Methyl-3-phenyl-2(3H)-thiazolon-hydrazon, 4-p-Biphenylyl-3-phenyl-2(3H)-thiazolon-hydrazon, 4-(4-Methoxy)phenyl-3-phenyl-2(3H)-thiazolon-hydrazon, 4-tert-Butyl-3-phenyl-2(3H)-thiazolon-hydrazon, 4,5-Dimethyl-3-phenyl-2(3H)-thiazolon-hydrazon, 5-Methyl-3,4-diphenyl-2(3H)-thiazolon-hydrazon, 3,4,5-Triphenyl-2(3H)-thiazolon-hydrazon, 4,5-Dimethyl-3-(phenylmethyl)-2(3H)-thiazolon-hydrazon, 3-(2-Propenyl)-2(3H)-thiazolon-hydrazon, 4-Methyl-3-(2-propenyl)-2(3H)-thiazolon-hydrazon, 4-tert-Butyl-3-(2-propenyl)-2(3H)-thiazolon-hydrazon, 4-Phenyl-3-(2-propenyl)-2(3H)-thiazolon-hydrazon, 4,5-Dimethyl-3-(2-propenyl)-2(3H)-thiazolon-hydrazon, 4,5-Diphenyl-3-(2-propenyl)-2(3H)-thiazolon-hydrazon, 2-Hydrazono-2,3-dihydro-3-[(phenylamino)carbonyl]-4-methyl-thiazolcarbonsäureethylester, 3-Methyl-4,5,6,7-tetrahydro-2(3H)-benzothiazolon-hydrazon, 3-Methyl-2(3H)-benzothiazolon-hydrazon, 3,6-Dimethyl-2(3H)-benzothiazolon-hydrazon, 6-Chlor-3-methyl-2(3H)-benzothiazolon-hydrazon, 7-Chlor-3-methyl-2(3H)-benzothiazolon-hydrazon, 6-Hydroxy-3-methyl-2(3H)-benzothiazolon-hydrazon, 5-Methoxy-3-methyl-2(3H)-benzothiazolon-hydrazon, 7-Methoxy-3-methyl-2(3H)-benzothiazolon-hydrazon, 5,6-Dimethoxy-3-methyl-2(3H)-benzothiazolon-hydrazon, 5-Ethoxy-3-methyl-2(3H)-benzothiazolon-hydrazon, 6-Ethoxy-3-methyl-2(3H)-benzothiazolon-hydrazon, 3-Methyl-5-nitro-2(3H)-benzothiazolon-hydrazon, 3-Methyl-6-nitro-2(3H)-benzothiazolon-hydrazon, 5-Acetamido-3-methyl-2(3H)-benzothiazolon-hydrazon, 6-Acetamido-3-methyl-2(3H)-benzothiazolon-hydrazon, 5-Anilino-3-methyl-2(3H)-benzothiazolon-hydrazon, 6-Anilino-3-methyl-2(3H)-benzothiazolon-hydrazon, 2-Hydrazono-2,3-dihydro-3-methyl-6-benzothiazol-carbonsäure, 2-Hydrazono-2,3-dihydro-3-methyl-4-benzothiazol-sulfonsäure, 2-Hydrazono-2,3-dihydro-3-methyl-5-benzothiazol-sulfonsäure, 2-Hydrazono-2,3-dihydro-3-methyl-6-benzothiazol-sulfonsäure, 2-Hydrazono-2,3-dihydro-3-methyl-7-benzothiazol-sulfonsäure, 2-Hydrazono-2,3-dihydro-N,N,3-trimethyl-6-benzothiazol-sulfonsäureamid, [(2-Hydrazono-2,3-dihydro-3-methyl-6-benzothiazolyl)oxy]essigsäure-hydrazid, 3-Methyl-naphtho[2,3-d]thiazol-2(3H)-on-hydrazon, 3-Ethyl-2(3H)-benzothiazolon-hydrazon, 6-Ethoxy-3-ethyl-2(3H)-benzothiazolon-hydrazon, 3-Propyl-2(3H)-benzothiazolon-hydrazon, 3-Butyl-2(3H)-benzothiazolon-hydrazon, 3-Hexyl-2(3H)-benzothiazolon-hydrazon, 3-Hydroxyethyl-2(3H)-benzothiazolon-hydrazon, 3-Aminoethyl-2(3H)-benzothiazolon-hydrazon, 3-p-Methylbenzyl-2(3H)-benzothiazolon-hydrazon, 2-Hydrazono-2,3-dihydro-3-(2-hydroxyethyl)-6-benzothiazol-carbonsäure, 2-Hydrazono-2,3-dihydro-6-methoxy-3(2H)-benzothiazol-propan-sulfonsäure, 6-Hexadecyloxy-2-hydrazono-3(2H)-benzothiazol-propan-sulfonsäure, 2-Oxo-3-benzothiazolin-essigsäureethylester-hydrazon, 3-Acetyl-2(3H)-benzothiazolon-hydrazon, 2-Hydrazono-3(2H)-benzothiazol-carboxaldehyd, 3-Methyl-2(3H)-oxazolon-hydrazon, 3-Phenyl-2(3H)-oxazolon-hydrazon, 3-Methyl-2(3H)-benzoxazolon-hydrazon, 3-Phenyl-2(3H)-benzoxazolon-hydrazon, N-Acetyl-3-methyl-2(3H)-thiazolon-hydrazon, N-Acetyl-3,4-dimethyl-2(3H)-thiazolon-hydrazon, N-Acetyl-3-methyl-4-phenyl-2(3H)-thiazolon-hydrazon, N-Acetyl-4-(4-methoxy)phenyl-3-methyl-2(3H)-thiazolon-hydrazon, N-Acetyl-3-methyl-4-(4-nitrophenyl)-2(3H)-thiazolon-hydrazon, N-Acetyl-4-([1,1'-biphenyl]-4-yl)-3-methyl-2(3H)-thiazolon-hydrazon, N-Acetyl-3-methyl-4-(2-naphtalenyl)-2(3H)-thiazolon-hydrazon, N-Acetyl-2-hydrazono-2,3-dihydro-3-methyl-4-thiazolcarbonsäure-ethylester, N-Acetyl-3,4,5-trimethyl-2(3H)-thiazolon-hydrazon, N-Acetyl-3,4-dimethyl-5-phenyl-2(3H)-thiazolon-hydrazon, N-Acetyl-3,5-dimethyl-4-phenyl-2(3H)-thiazolon-hydrazon, N-Acetyl-3-methyl-4,5-diphenyl-2(3H)-thiazolon-hydrazon, N-Acetyl-3-ethyl-4,5-dimethyl2(3H)-thiazolon-hydrazon, N-Acetyl-4-methyl-3-phenyl-2(3H)-thiazolon-hydrazon, N-Acetyl-3,4-diphenyl-2(3H)-thiazolon-hydrazon, N-Acetyl-4-p-biphenylyl-3-phenyl-2(3H)-thiazolon-hydrazon, N-Acetyl-4-(4-methoxy)phenyl-3-phenyl-2(3H)-thiazolon-hydrazon, N-Acetyl-4-tert-butyl-3-phenyl-2(3H)-thiazolon-hydrazon, N-Acetyl-4,5-dimethyl-3-phenyl-2(3H)-thiazolon-hydrazon, N-Acetyl-5-methyl-3,4-diphenyl-2(3H)-thiazolon-hydrazon, N-Acetyl-3,4,5-triphenyl-2(3H)-thiazolon-hydrazon, N-Acetyl-3-methyl-2(3H)-benzothiazolon-hydrazon, N-Acetyl-3-ethyl-2(3H)-benzothiazolon-hydrazon, N-Acetyl-3-butyl-2(3H)-benzothiazolon-hydrazon, N-Acetyl-3-hexyl-2(3H)-benzo-thiazolon-hydrazon, N-Acetyl-3-p-methylbenzyl-2(3H)-benzothiazolon-hydrazon, N-Acetyl-3-methyl-2(3H)-oxazolon-hydrazon, N-Acetyl-3-phenyl-2(3H)-oxazolon-hydrazon, N-Acetyl-3-methyl-2(3H)-benzoxazolon-hydrazon, N-Acetyl-3-phenyl-2(3H)-benzoxazolon-hydrazon, N-Formyl-3-methyl-2(3H)-thiazolon-hydrazon, N-Formyl-3,4-dimethyl-2(3H)-thiazolon-hydrazon, N-Formyl-3-methyl-4-phenyl-2(3H)-thiazolon-hydrazon, N-Formyl-4-(4-methoxy)phenyl-3-methyl-2(3H)-thiazolon-hydrazon, N-Formyl-3-methyl-4-(4-nitrophenyl)-2(3H)-thiazolon-hydrazon, N-Formyl-4-([1,1'-biphenyl]-4-yl)-3-methyl-2(3H)-thiazolon-hydrazon, N-Formyl-3-methyl-4-(2-naphtalenyl)-2(3H)-thiazolon-hydrazon, N-Formyl-2-hydrazono-2,3-dihydro-3-methyl-4-thiazolcarbonsäure-ethylester, N-Formyl-3,4,5-trimethyl-2(3H)-thiazolon-hydrazon, N-Formyl-3,4-dimethyl-5-phenyl-2(3H)-thiazolon-hydrazon, N-Formyl-3,5-dimethyl-4-phenyl-2(3H)-thiazolon-hydrazon, N-Formyl-3-methyl-4,5-diphenyl-2(3H)-thiazolon-hydrazon, N-Formyl-3-ethyl-4,5-dimethyl-2(3H)-thiazolon-hydrazon, N-Formyl-4-methyl-3-phenyl-2(3H)-thiazolon-hydrazon, N-Formyl-3,4-diphenyl-2(3H)-thiazolon-hydrazon, N-Formyl-4-p-biphenylyl-3-phenyl-2(3H)-thiazolon-hydrazon, N-Formyl-4-(4-methoxy)phenyl-3-phenyl-2(3H)-thiazolon-hydrazon, N-Formyl-4-tert-butyl-3-phenyl-2(3H)-thiazolon-hydrazon, N-Formyl-4,5-dimethyl-3-phenyl-2(3H)-thiazolon-hydrazon, N-Formyl-5-methyl-3,4-diphenyl-2(3H)-thiazolon-hydrazon, N-Formyl-3,4,5-triphenyl-2(3H)-thiazolon-hydrazon, N-Formyl-3-methyl-2(3H)-benzothiazolon-hydrazon, N-Formyl-3-ethyl-2(3H)-benzothiazolon-hydrazon, N-Formyl-3-butyl-2(3H)-benzothiazolon-hydrazon, N-Formyl-3-hexyl-2(3H)-benzothiazolon-hydrazon, N-Formyl-3-p-methylbenzyl-2(3H)-benzothiazolon-hydrazon, N-Formyl-3-methyl-2(3H)-oxazolon-hydrazon, N-Formyl-3-phenyl-2(3H)-oxazolon-hydrazon, N-Formyl-3-methyl-2(3H)-benzoxazolon-hydrazon und N-Formyl-3-phenyl-2(3H)-benzoxazolon-hydrazon.

4. Mittel nach einem der Ansprüche 1 bis 3, **dadurch gekennzeichnet, dass** die Kupplersubstanz ausgewählt ist aus N-(3-Dimethylaminophenyl)-harnstoff, 2,6-Diamino-pyridin, 2-Amino-4-[(2-hydroxyethyl)-amino]-anisol, 2,4-Diamino-1-fluor-5-methyl-benzol, 2,4-Diamino-1-methoxy-5-methyl-benzol, 2,4-Diamino-1-ethoxy-5-methyl-benzol, 2,4-Diamino-1-(2-hydroxy-ethoxy)-5-methyl-benzol, 2,4-Di[(2-hydroxyethyl)amino]-1,5-dimethoxy-benzol, 2,3-Diamino-6-methoxy-pyridin, 3-Amino-6-methoxy-2-(methyl-amino)-pyridin, 2,6-Diamino-3,5-dimethoxy-pyridin, 3,5-Diamino-2,6-dimethoxy-pyridin, 1,3-Diamino-benzol, 2,4-Diamino-1-(2-hydroxyethoxy)-benzol, 1,3-Diamino-4-(2,3-dihydroxypropoxy)-benzol, 1,3-Diamino-4-(3-hydroxypropoxy)-benzol, 1,3-Diamino-4-(2-methoxyethoxy)-benzol, 2,4-Diamino-1,5-di(2-hydroxyethoxy)-benzol, 1-(2-Aminoethoxy)-2,4-diamino-benzol, 2-Amino-1-(2-hydroxyethoxy)-4-methylamino-benzol, 2,4-Diaminophenoxyessigsäure, 3-[Di(2-hydroxyethyl)amino]-anilin, 4-Amino-2-di[(2-hydroxyethyl)amino]-1-ethoxy-benzol, 5-Methyl-2-(1-methylethyl)-phenol, 3-[(2-Hydroxyethyl)amino]-anilin, 3-[(2-Aminoethyl)-amino]-anilin, 1,3-Di(2,4-diaminophenoxy)-propan, Di(2,4-diamino-phenoxy)-methan, 1,3-Diamino-2,4-dimethoxy-benzol, 2,6-Bis(2-hydroxyethyl)amino-toluol, 4-Hydroxyindol, 3-Dimethylamino-phenol, 3-Diethylamino-phenol, 5-Amino-2-methyl-phenol, 5-Amino-4-fluor-2-methyl-phenol, 5-Amino-4-methoxy-2-methyl-phenol, 5-Amino-4-ethoxy-2-methyl-phenol, 3-Amino-2,4-dichlor-phenol, 5-Amino-2,4-dichlor-phenol, 3-Amino-2-methyl-phenol, 3-Amino-2-chlor-6-methyl-phenol, 3-Amino-phenol, 2-[(3-Hydroxyphenyl)-amino]-acetamid, 5-[(2-Hydroxyethyl)amino]-4-methoxy-2-methyl-phenol, 5-[(2-Hydroxyethyl)amino]-2-methyl-phenol, 3-[(2-Hydroxyethyl)amino]-phenol, 3-[(2-Methoxyethyl)amino]-phenol, 5-Amino-2-ethyl-phenol, 5-Amino-2-methoxy-phenol, 2-(4-Amino-2-hydroxyphenoxy)-ethanol, 5-[(3-Hydroxypropyl)amino]-2-methyl-phenol, 3-[(2,3-Dihydroxypropyl)-amino]-2-methyl-phenol, 3-[(2-Hydroxyethyl)-amino]-2-methyl-phenol, 2-Amino-3-hydroxy-pyridin, 2,6-Dihydroxy-3,4-dimethylpyridin, 5-Amino-4-chlor-2-methyl-phenol, 1-Naphthol, 2-Methyl-1-naphthol, 1,5-Dihydroxy-naphthalin, 1,7-Dihydroxy-naphthalin, 2,3-Dihydroxy-naphthalin, 2,7-Dihydroxy-naphthalin, 2-Methyl-1-naphthol-acetat, 1,3-Dihydroxybenzol, 1-Chlor-2,4-dihydroxy-benzol, 2-Chlor-1,3-dihydroxy-benzol, 1,2-Dichlor-3,5-dihydroxy-4-methyl-benzol, 1,5-Dichlor-2,4-dihydroxybenzol, 1,3-Dihydroxy-2-methyl-benzol, 3,4-Methylendioxy-phenol, 3,4-Methylendioxy-anilin, 5-[(2-Hydroxyethyl)amino]-1,3-benzo-dioxol, 6-Brom-1-hydroxy-3,4-methylendioxy-benzol, 3,4-Diamino-benzoesäure, 3,4-Dihydro-6-hydroxy-9,4(2H)-benzoxazin, 6-Amino-3,4-dihydro-1,4(2H)-benzoxazin, 3-Methyl-1-phenyl-5-pyrazolon, 5,6-Dihydroxy-indol, 5,6-Dihydroxy-indolin, 5-Hydroxy-indol, 6-Hydroxy-indol, 7-Hydroxy-indol und 2,3-Indolindion.

5. Mittel nach einem der Ansprüche 1 bis 4, **dadurch gekennzeichnet, dass** das Persulfatsalz ausgewählt ist aus Kaliumpersulfat, Natriumpersulfat und Ammoniumpersulfat.

6. Mittel nach einem der Ansprüche 1 bis 5, **dadurch gekennzeichnet, dass** es die Hydrazon-Derivate der Formel (I)*,* sowie die Kupplersubstanzen und die Persulfatsalze jeweils in einer Gesamtmenge von 0,01 bis 10 Gewichtsprozent enthält.

7. Mittel nach einem der Ansprüche 1 bis 5, **dadurch gekennzeichnet, dass** es zusätzlich 0,01 bis 10 Gewichtsprozent eines physiologisch unbedenklichen, direktziehenden Farbstoffs aus der Gruppe der kationischen und anionischen Farbstoffe, der Dispersionsfarbstoffe, der Nitrofarbstoffe, der Azofarbstoffe, der Chinonfarbstoffe und der Triphenylmethanfarbstoffe enthält.

8. Mittel nach einem der Ansprüche 1 bis 7, **dadurch gekennzeichnet, dass** es einen pH-Wert von 3 bis 10 aufweist.

9. Mittel nach einem der Ansprüche 1 bis 8, **dadurch gekennzeichnet, dass** es ein Haarfärbemittel ist.

10. 2-Komponenten-Kit zur Färbung von Keratinfasern bestehend aus einer Farbträgermasse (A1), welche die Verbindung der Formel (I) enthält, und einer weiteren Farbträgermasse (A2), welche die Kupplersubstanzen und die Persulfatsalze enthält, sowie gegebenenfalls einem Mittel zur Einstellung des pH-Wertes.

11. 2-Komponenten-Kit zur Färbung von keratinfasern dessen 1. Komponente aus einem die Verbindungen der Formel (I), die Kupplersubstanzen und die Persulfatsalze sowie gegebenenfalls weitere übliche pulverförmige kosmetische Zusatzstoffe enthaltenden Pulver besteht, und dessen 2. Komponente Wasser oder eine flüssige kosmetische Zubereitung ist, welche gegebenenfalls ein Mittel zur Einstellung des pH-Wertes enthält.

12. Verfahren zum Färben von Haaren bei dem ein Färbemittel nach einem der Ansprüche 1 bis 9 auf die Haare aufgetragen wird und nach einer Einwirkungszeit von 5 bis 60 Minuten bei einer Temperatur von 20 bis 50 °C das Haar mit Wasser gespült, gegebenenfalls mit einem Shampoo gewaschen und sodann getrocknet wird.

13. Verfahren zum Färben von Haaren bei dem das gebrauchsfertige Färbemittel (A) unmittelbar vor der Anwendung durch Vermischen zweier Komponenten (A1) und (A2) -gegebenenfalls unter Zusatz eines Alkalisierungsmittels oder einer Säure- hergestellt und sodann auf die Haare aufgetragen wird und nach einer Einwirkungszeit von 5 bis 60 Minuten bei einer Temperatur von 20 bis 50 °C das Haar mit Wasser gespült, gegebenenfalls mit einem Shampoo gewaschen und sodann getrocknet wird, **dadurch gekennzeichnet, dass** ein durch Vermischen zweier Komponenten (A1) und (A2) erhältliches Färbemittel (A) gemäß Anspruch 10 oder 11 verwendet wird.

## Claims

1. Agent for dyeing keratin fibres (A), **characterized in that** it comprises (a) at least one hydrazone derivative of the formula (I) or physiologically compatible salt thereof, 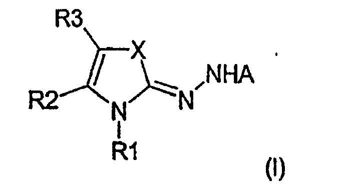 in which **X** is oxygen or sulphur;
**A** is hydrogen, an acetyl group, a trifluoroacetyl group, a formyl group, a (C₁-C₆)-alkylsulphonyl group or an arylsulphonyl group;
**R1** is a saturated or unsaturated (C₁-C₁₂)-alkyl group, a (C₁-C₁₂)-alkyl group substituted by a halogen atom (F, Cl, Br, I), a hydroxy- (C₁-C₁₂)-alkyl group, an amino (C₁-C₁₂)-alkyl group, a sulpho- (C₁-C₁₂)-alkyl group, a formyl group, a C(O)-(C₁-C₁₂)-alkyl group, a C (O) -phenyl group, a C(O)NH-(C₁-C₁₂)-alkyl group, a C(O)NH-phenyl group, a substituted or unsubstituted phenyl group, or a benzyl group;
**R2** and **R3** may be identical or different and, independently of one another, are hydrogen, a halogen atom (F, Cl, Br, I), a saturated or unsaturated (C₁-C₁₂)-alkyl group, a (C₁-C₁₂)-alkyl group substituted by a halogen atom (F, Cl, Br, I), a hydroxy-(C₁-C₁₂)-alkyl group, a (C₁-C₁₂)-alkoxy group, a cyano group, a nitro group, an amino group, a (C₁-C₁₂)-alkylamino group, a (C₁-C₁₂)-dialkylamino group, a carboxylic acid, a C (O) O- (C₁-C₁₂) -alkyl group, a substituted or unsubstituted C(O)O-phenyl group, a substituted or unsubstituted phenyl group or a naphthyl group,
or **R2** and **R3**, together with the remainder of the molecule, form a heterocyclic or carbocyclic, saturated or unsaturated, substituted or unsubstituted ring system;
(b) at least one coupler substance known per se or physiologically compatible salt thereof,
and (c) as oxidative agent, a persulphate salt.

2. Agent according to Claim 1, **characterized in that X** is sulphur, **A** is hydrogen, **R1** is a saturated or unsaturated (C₁-C₁₂)-alkyl group, a hydroxy (C₁-C₁₂)-alkyl group, an amino (C₁-C₁₂) -alkyl group, or a substituted or unsubstituted phenyl group, and **R2** and **R3**, independently of one another, are hydrogen, a saturated or unsaturated (C₁-C₁₂)-alkyl group, a cyano group, a nitro group, an amino group, a (C₁-C₁₂)-alkylamino group, a (C₁-C₁₂)-dialkylamino group, a C(O)O-alkyl group or a substituted or unsubstituted phenyl group or a naphthyl group, or **R2** and **R3**, together with the remainder of the molecule, form a carbocyclic, unsaturated, substituted or unsubstituted ring system.

3. Agent according to Claim 1 or 2, **characterized in that** the hydrazone derivative of the formula (I) is chosen from 3-methyl-2(3H)-thiazolone hydrazone, 3,4-dimethyl-2(3H)-thiazolone hydrazone, 4-tert-butyl-3-methyl-2(3H)-thiazolone hydrazone, 3-methyl-4-phenyl-2(3H)-thiazolone hydrazone, 3-methyl-4-(4-tolyl)-2(3H)-thiazolone hydrazone, 4-(4-methoxy)phenyl-3-methyl-2(3H)-thiazolone hydrazone, 4-(4-ethoxy)phenyl-3-methyl-2(3H)-thiazolone hydrazone, 4-(4-bromophenyl)-3-methyl-2(3H)-thiazolone hydrazone, 4-(3-bromophenyl)-3-methyl-2(3H)-thiazolone hydrazone, 4-(4-chlorophenyl)-3-methyl-2(3H)-thiazolone hydrazone, 4-(3-chlorophenyl)-3-methyl-2(3H)-thiazolone hydrazone, 3-methyl-4-(4-nitrophenyl)-2(3H)-thiazolone hydrazone, 3-methyl-4-(3-nitrophenyl)-2(3H)-thiazolone hydrazone, 4-([1,1'-biphenyl]-4-yl)-3-methyl-2(3H)-thiazolone hydrazone, 3-methyl-4-(2-naphthalenyl)-2(3H)-thiazolone hydrazone, 2-hydrazono-2,3-dihydro-3-methyl-4-thiazolecarboxylic ethyl ester, 3,4,5-trimethyl-2(3H)-thiazolone hydrazone, 3,4-dimethyl-5-phenyl-2(3H)-thiazolone hydrazone, 3,5-dimethyl-4-phenyl-2(3H)-thiazolone hydrazone, 3-methyl-4,5-diphenyl-2(3H)-thiazolone hydrazone, 5-ethyl-3-methyl-4-phenyl-2(3H)-thiazolone hydrazone, 4-(4-bromophenyl)-3-methyl-5-phenyl-2(3H)-thiazolone hydrazone, 3-methyl-5-phenyl-4-(4-tolyl)-2(3H)-thiazolone hydrazone, 5-(4-chlorophenyl)-4-phenyl-3-methyl-2(3H)-thiazolone hydrazone, 5-(4-chlorophenyl)-4-(4-methoxyphenyl)-3-methyl-2(3H)-thiazolone hydrazone, 2-hydrazono-2,3-dihydro-3,4-dimethyl-4-thiazolecarboxylic ethyl ester, 4-amino-2-hydrazono-2,3-dihydro-3-methyl-5-thiazolecarbonitrile, 3-ethyl-4,5-dimethyl-2(3H)-thiazolone hydrazone, 2-hydrazono-2,3-dihydro-3-ethyl-4-methylthiazolecarboxylic ethyl ester, 5-methyl-3-(1-methylethyl)-4-phenyl-2(3H)-thiazolone hydrazone, 4,5-dimethyl-3-(1-methylethyl)-2(3H)-thiazolone hydrazone, 3-(1-methylethyl)-4,5-diphenyl-2(3H)-thiazolone hydrazone, 4,5-dimethyl-3-propyl-2(3H)-thiazolone hydrazone, 4,5-diphenyl-3-propyl-2(3H)-thiazolone hydrazone, 3-butyl-4,5-diphenyl-2(3H)-thiazolone hydrazone, 4,5-dimethyl-3-(2-methylpropyl)-2(3H)-thiazolone hydrazone, 3-(2-methylpropyl)-4,5-diphenyl-2(3H)-thiazolone hydrazone, 3-hydroxyethyl-2(3H)-thiazolone hydrazone, 3-hydroxyethyl-4-methyl-2(3H)-thiazolone hydrazone, 3-hydroxyethyl-4,5-dimethyl-2(3H)-thiazolone hydrazone, 3-aminoethyl-2(3H)-thiazolone hydrazone, 3-aminoethyl-4-methyl-2(3H)-thiazolone hydrazone, 3-aminoethyl-4,5-dimethyl-2(3H)-thiazolone hydrazone, 3,4-diphenyl-2(3H)-thiazolone hydrazone, 4-methyl-3-phenyl-2(3H)-thiazolone hydrazone, 4-p-biphenylyl-3-phenyl-2(3H)-thiazolone hydrazone, 4-(4-methoxy)phenyl-3-phenyl-2(3H)-thiazolone hydrazone, 4-tert-butyl-3-phenyl-2(3H)-thiazolone hydrazone, 4,5-dimethyl-3-phenyl-2(3H)-thiazolone hydrazone, 5-methyl-3,4-diphenyl-2(3H)-thiazolone hydrazone, 3,4,5-triphenyl-2(3H)-thiazolone hydrazone, 4,5-dimethyl-3-(phenylmethyl)-2(3H)-thiazolone hydrazone, 3-(2-propenyl)-2(3H)-thiazolone hydrazone, 4-methyl-3-(2-propenyl)-2(3H)-thiazolone hydrazone, 4-tert-butyl-3-(2-propenyl)-2(3H)-thiazolone hydrazone, 4-phenyl-3-(2-propenyl)-2(3H)-thiazolone hydrazone, 4,5-dimethyl-3-(2-propenyl)-2(3H)-thiazolone hydrazone, 4,5-diphenyl-3(2-propenyl)-2(3H)-thiazolone hydrazone, 2-hydrazono-2,3-dihydro-3-[(phenylamino)carbonyl]-4-methylthiazolecarboxylic ethyl ester, 3-methyl-4,5,6,7-tetrahydro-2(3H)-benzothiazolone hydrazone, 3-methyl-2(3H)-benzothiazolone hydrazone, 3,6-dimethyl-2(3H)-benzothiazolone hydrazone, 6-chloro-3-methyl-2(3H)-benzothiazolone hydrazone, 7-chloro-3-methyl-2(3H)-benzothiazolone hydrazone, 6-hydroxy-3-methyl-2(3H)-benzothiazolone hydrazone, 5-methoxy-3-methyl-2(3H)-benzothiazolone hydrazone, 7-methoxy-3-methyl-2(3H)-benzothiazolone hydrazone, 5,6-dimethoxy-3-methyl-2(3H)-benzothiazolone hydrazone, 5-ethoxy-3-methyl-2(3H)-benzothiazolone hydrazone, 6-ethoxy-3-methyl-2(3H)-benzothiazolone hydrazone, 3-methyl-5-nitro-2(3H)-benzothiazolone hydrazone, 3-methyl-6-nitro-2(3H)-benzothiazolone hydrazone, 5-acetamido-3-methyl-2(3H)-benzothiazolone hydrazone, 6-acetamido-3-methyl-2(3H)-benzothiazolone hydrazone, 5-anilino-3-methyl-2(3H)-benzothiazolone hydrazone, 6-anilino-3-methyl-2(3H)-benzothiazolone hydrazone, 2-hydrazono-2,3-dihydro-3-methyl-6-benzothiazolecarboxylic acid, 2-hydrazono-2,3-dihydro-3-methyl-4-benzothiazolesulphonic acid, 2-hydrazono-2,3-dihydro-3-methyl-5-benzothiazolesulphonic acid, 2-hydrazono-2,3-dihydro-3-methyl-6-benzothiazolesulphonic acid, 2-hydrazono-2,3-dihydro-3-methyl-7-benzothiazolesulphonic acid, 2-hydrazono-2,3-dihydro-N,N,3-trimethyl-6-benzothiazolesulphonamide, [(2-hydrazono-2,3-dihydro-3-methyl-6-benzothiazolyl)oxy]acetic hydrazide, [(2-hydrazono-2,3-dihydro-3-methyl-6-benzothiazolyl)oxy]-acetic hydrazide, 3-methylnaphtho[2,3-d]thiazol-2(3H)-one hydrazone, 3-ethyl-2(3H)-benzothiazolone hydrazone, 6-ethoxy-3-ethyl-2(3H)-benzothiazolone hydrazone, 3-propyl-2(3H)-benzothiazolone hydrazone, 3-butyl-2(3H)-benzothiazolone hydrazone, 3-hexyl-2(3H)-benzothiazolone hydrazone, 3-hydroxyethyl-2(3H)-benzothiazolone hydrazone, 3-aminoethyl-2(3H)-benzothiazolone hydrazone, 3-p-methylbenzyl-2(3H)-benzothiazolone hydrazone, 2-hydrazono-2,3-dihydro-3-(2-hydroxyethyl)-6-benzothiazolecarboxylic acid, 2-hydrazono-2,3-dihydro-6-methoxy-3(2H)-benzothiazole-propanesulphonic acid, 6-hexadecyloxy-2-hydrazono-3(2H)-benzothiazolepropanesulphonic acid, 2-oxo-3-benzothiazolineacetic ethyl ester hydrazone, 3-acetyl-2(3H)-benzothiazolone hydrazone, 2-hydrazono-3(2H)-benzothiazolecarboxaldehyde, 3-methyl-2(3H)-oxazolone hydrazone, 3-phenyl-2(3H)-oxazolone hydrazone, 3-methyl-2(3H)-benzoxazolone hydrazone, 3-phenyl-2(3H)-benzoxazolone hydrazone, N-acetyl-3-methyl-2(3H)-thiazolone hydrazone, N-acetyl-3,4-dimethyl-2(3H)-thiazolone hydrazone, N-acetyl-3-methyl-4-phenyl-2(3H)-thiazolone hydrazone, N-acetyl-4-(4-methoxy)phenyl-3-methyl-2(3H)-thiazolone hydrazone, N-acetyl-3-methyl-4-(4-nitrophenyl)-2(3H)-thiazolone hydrazone, N-acetyl-4-([1,1'-biphenyl]-4-yl)-3-methyl-2(3H)-thiazolone hydrazone, N-acetyl-3-methyl-4-(2-naphthalenyl)-2(3H)-thiazolone hydrazone, N-acetyl-2-hydrazono-2,3-dihydro-3-methyl-4-thiazolecarboxylic ethyl ester, N-acetyl-3,4,5-trimethyl-2(3H)-thiazolone hydrazone, N-acetyl-3,4-dimethyl-5-phenyl-2(3H)-thiazolone hydrazone, N-acetyl-3,4-dimethyl-4-phenyl-2(3H)-thiazolone hydrazone, N-acetyl-3-methyl-4,5-diphenyl-2(3H)-thiazolone hydrazone, N-acetyl-3-ethyl-4,5-dimethyl-2(3H)-thiazolone hydrazone, N-acetyl-4-methyl-3-phenyl-2(3H)-thiazolone hydrazone, N-acetyl-3,4-diphenyl-2(3H)-thiazolone hydrazone, N-acetyl-4-p-biphenylyl-3-phenyl-2(3H)-thiazolone hydrazone, N-acetyl-4-(4-methoxy)phenyl-3-phenyl-2(3H)-thiazolone hydrazone, N-acetyl-4-tert-butyl-3-phenyl-2(3H)-thiazolone hydrazone, N-acetyl-4,5-dimethyl-3-phenyl-2(3H)-thiazolone hydrazone, N-acetyl-5-methyl-3,4-diphenyl-2(3H)-thiazolone hydrazone, N-acetyl-3,4,5-triphenyl-2(3H)-thiazolone hydrazone, N-acetyl-3-methyl-2(3H)-benzothiazolone hydrazone, N-acetyl-3-ethyl-2(3H)-benzothiazolone hydrazone, N-acetyl-3-butyl-2(3H)-benzothiazolone hydrazone, N-acetyl-3-hexyl-2(3H)-benzothiazolone hydrazone, N-acetyl-3-p-methylbenzyl-2(3H)-benzothiazolone hydrazone, N-acetyl-3-methyl-2(3H)-oxazolone hydrazone, N-acetyl-3-phenyl-2(3H)-oxazolone hydrazone, N-acetyl-3-methyl-2(3H)-benzoxazolone hydrazone, N-acetyl-3-phenyl-2(3H)-benzoxazolone hydrazone, N-formyl-3-methyl-2(3H)-thiazolone hydrazone, N-formyl-3,4-dimethyl-2(3H)-thiazolone hydrazone, N-formyl-3-methyl-4-phenyl-2(3H)-thiazolone hydrazone, N-formyl-4-(4-methoxy)phenyl-3-methyl-2(3H)-thiazolone hydrazone, N-formyl-3-methyl-4-(4-nitrophenyl)-2(3H)-thiazolone hydrazone, N-formyl-4-([1,1'-biphenyl]-4-yl)-3-methyl-2(3H)-thiazolone hydrazone, N-formyl-3-methyl-4(2-naphthalenyl)-2(3H)-thiazolone hydrazone, N-formyl-2-hydrazono-2,3-dihydro-3-methyl-4-thiazolecarboxylic ethyl ester, N-formyl-3,4,5-trimethyl-2(3H)-thiazolone hydrazone, N-formyl-3,4-dimethyl-5-phenyl-2(3H)-thiazolone hydrazone, N-formyl-3,5-dimethyl-4-phenyl-2(3H)-thiazolone hydrazone, N-formyl-3-methyl-4,5-diphenyl-2(3H)-thiazolone hydrazone, N-formyl-3-ethyl-4,5-dimethyl-2(3H)-thiazolone hydrazone, N-formyl-4-methyl-3-phenyl-2(3H)-thiazolone hydrazone, N-formyl-3,4-diphenyl-2(3H)-thiazolone hydrazone, N-formyl-4-p-biphenylyl-3-phenyl-2(3H)-thiazolone hydrazone, N-formyl-4-(4-methoxy)phenyl-3-phenyl-2(3H)-thiazolone hydrazone, N-formyl-4-tert-butyl-3-phenyl-2(3H)-thiazolone hydrazone, N-formyl-4,5-dimethyl-3-phenyl-2(3H)-thiazolone hydrazone, N-formyl-5-methyl-3,4-diphenyl-2(3H)-thiazolone hydrazone, N-formyl-3,4,5-triphenyl-2(3H)-thiazolone hydrazone, N-formyl-3-methyl-2(3H)-benzothiazolone hydrazone, N-formyl-3-ethyl-2(3H)-benzothiazolone hydrazone, N-formyl-3-butyl-2(3H)-benzothiazolone hydrazone, N-formyl-3-hexyl-2(3H)-benzothiazolone hydrazone, N-formyl-3-p-methylbenzyl-2(3H)-benzothiazolone hydrazone, N-formyl-3-methyl-2(3H)-oxazolone hydrazone, N-formyl-3-phenyl-2(3H)-oxazolone hydrazone, N-formyl-3-methyl-2(3H)-benzoxazolone hydrazone and N-formyl-3-phenyl-2(3H)-benzoxazolone hydrazone.

4. Agent according to one of Claims 1 to 3, **characterized in that** the coupler substance is chosen from N-(3-dimethylaminophenyl)urea, 2,6-diaminopyridine, 2-amino-4-[(2-hydroxyethyl)amino]anisole, 2,4-diamino-1-fluoro-5-methylbenzene, 2,4-diamino-1-methoxy-5-methylbenzene, 2,4-diamino-1-ethoxy-5-methylbenzene, 2,4-diamino-1-(2-hydroxyethoxy)-5-methylbenzene, 2,4-di[(2-hydroxyethyl)amino]-1,5-dimethoxybenzene, 2,3-diamino-6-methoxypyridine, 3-amino-6-methoxy-2-(methylamino)pyridine, 2,6-diamino-3,5-di-methoxypyridine, 3,5-diamino-2,6-dimethoxypyridine, 1,3-diaminobenzene, 2,4-diamino-1-(2-hydroxyethoxy)-benzene, 1,3-diamino-4-(2,3-dihydroxypropoxy)benzene, 1,3-diamino-4-(3-hydroxypropoxy)benzene, 1,3-diamino-4-(2-methoxyethoxy)benzene, 2,4-diamino-1,5-di(2-hydroxyethoxy)benzene, 1-(2-aminoethoxy)-2,4-diaminobenzene, 2-amino-1-(2-hydroxyethoxy)-4-methylaminobenzene, 2,4-diaminophenoxyacetic acid, 3[[di-(2-hydroxyethyl)-amino]aniline, 4-amino-2-di-[(2-hydroxyethyl)amino]-1-ethoxybenzene, 5-methyl-2-(1-methylethyl)phenol, 3-[(2-hydroxyethyl)amino]aniline, 3-[(2-aminoethyl)amino]-aniline, 1,3-di-(2,4-diaminophenoxy)propane, di-(2,4-diaminophenoxy)methane, 1,3-diamino-2,4-dimethoxybenzene, 2,6-bis-(2-hydroxyethyl)aminotoluene, 4-hydroxyindole, 3-dimethylaminophenol, 3-diethylaminophenol, 5-amino-2-methylphenol, 5-amino-4-fluoro-2-methylphenol, 5-amino-4-methoxy-2-methylphenol, 5-amino-4-ethoxy-2-methylphenol, 3-amino-2,4-dichlorophenol, 5-amino-2,4-dichlorophenol, 3-amino-2-methyl-phenol, 3-amino-2-chloro-6-methylphenol, 3-aminophenol, 2-[(3-hydroxyphenyl)amino]acetamide, 5-[(2-hydroxyethyl)amino]-4-methoxy-2-methylphenol, 5-[(2-hydroxyethyl)amino]-2-methylphenol, 3-[(2-hydroxyethyl)amino]-phenol, 3-[(2-methoxyethyl)amino]phenol, 5-amino-2-ethylphenol, 5-amino-2-methoxyphenol, 2-(4-amino-2-hydroxyphenoxy)ethanol, 5-[(3-hydroxypropyl)amino]-2-methylphenol, 3-[(2,3-dihydroxypropyl)amino]-2-methyl-phenol, 3-[(2-hydroxyethyl)amino]-2-methylphenol, 2-amino-3-hydroxypyridine, 2,6-dihydroxy-3,4-dimethylpyridine, 5-amino-4-chloro-2-methylphenol, 1-naphthol, 2-methyl-1-naphthol, 1,5-dihydroxynaphthalene, 1,7-dihydroxynaphthalene, 2,3-dihydroxynaphthalene, 2,7-dihydroxynaphthalene, 2-methyl-1-naphthol acetate, 1,3-dihydroxybenzene, 1-chloro-2,4-dihydroxybenzene, 2-chloro-1,3-dihydroxybenzene, 1,2-dichloro-3,5-dihydroxy-4-methylbenzene, 1,5-dichloro-2,4-dihydroxybenzene, 1,3-dihydroxy-2-methylbenzene, 3,4-methylene-dioxyphenol, 3,4-methylenedioxyaniline, 5-[(2-hydroxyethyl)amino]-1,3-benzodioxole, 6-bromo-1-hydroxy-3,4-methylenedioxybenzene, 3,4-diaminobenzoic acid, 3,4-dihydro-6-hydroxy-1,4(2H)-benzoxazine, 6-amino-3,4-dihydro-1,4(2H)-benzoxazine, 3-methyl-1-phenyl-5-pyrazolone, 5,6-dihydroxyindole, 5,6-dihydroxyindoline, 5-hydroxyindole, 6-hydroxyindole, 7-hydroxyindole and 2,3-indolinedione.

5. Agent according to one of Claims 1 to 4, **characterized in that** the persulphate salt is chosen from potassium persulphate, sodium persulphate and ammonium persulphate.

6. Agent according to one of Claims 1 to 5, **characterized in that** it comprises the hydrazone derivative of the formula (I), and the coupler substances and the persulphate salts, in each case in a total amount of from 0.01 to 10 per cent by weight.

7. Agent according to one of Claims 1 to 5, **characterized in that** it additionally comprises 0.01 to 10 per cent by weight of a physiologically acceptable, direct dye from the group of cationic and anionic dyes, disperse dyes, nitro dyes, azo dyes, quinone dyes and triphenylmethane dyes.

8. Agent according to one of Claims 1 to 7, **characterized in that** it has a pH of from 3 to 10.

9. Agent according to one of Claims 1 to 8, **characterized in that** it is a hair colourant.

10. Two-component kit for dyeing keratin fibres, consisting of a colour carrier mass (A1) which comprises the compound of the formula (I), and a further colour carrier mass (A2) which comprises the coupler substances and the persulphate salts, and optionally an agent for adjusting the pH.

11. Two-component kit for dyeing keratin fibres, whose first component consists of a powder comprising the compounds of the formula (I), the coupler substances and the persulphate salts, and optionally further customary pulverulent cosmetic additives, and whose second component is water or a liquid cosmetic preparation which optionally comprises an agent for adjusting the pH.

12. Method for dyeing hair in which a colourant according to one of Claims 1 to 9 is applied to the hair and, after a contact time of from 5 to 60 minutes at a temperature of from 20 to 50°C, the hair is rinsed with water, optionally washed with a shampoo and then dried.

13. Method for dyeing hair in which the ready-to-use colourant (A) is prepared directly prior to use by mixing two components (A1) and (A2) - optionally with the addition of an alkalizing agent or an acid - and then applied to the hair and, after a contact time of from 5 to 60 minutes at a temperature of from 20 to 50°C, the hair is rinsed with water, optionally washed with a shampoo and then dried, **characterized in that** a colourant (A) according to Claim 10 or 11 obtainable by mixing two components (A1) and (A2) is used.

## Revendications

1. Composition (A) pour la teinture de fibres de kératine, **caractérisée en ce qu'**elle contient (a) au moins un dérivé d'hydrazine de formule (I) ou un sel physiologiquement acceptable d'un tel dérivé, 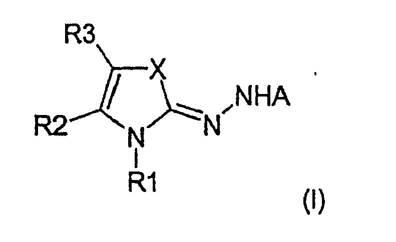 formule dans laquelle **X** est un atome d'oxygène ou de soufre ;
**A** représente un atome d'hydrogène, un groupe acétyle, un groupe trifluoroacétyle, un groupe formyle, un groupe alkyl(C₁-C₆)sulfonyle ou un groupe arylsulfonyle;
**R1** est un groupe alkyle en C₁-C₁₂ saturé ou insaturé, un groupe alkyle en C₁-C₁₂ substitué par un atome d'halogène (F, Cl, Br, I), un groupe hydroxyalkyle(C₁-C₁₂), un groupe aminoalkyle-(C₁-C₁₂), un groupe sulfoalkyle (C₁-C₁₂), un groupe formyle, un groupe C(O)-alkyle (C₁-C₁₂), un groupe C(O)-phényle, un groupe C(O)NH-alkyle(C₁-C₁₂), un groupe C(O)NH-phényle, un groupe phényle substitué ou non substitué ou un groupe benzyle ;
**R2** et **R3** peuvent être identiques ou différents et représentent, indépendamment l'un de l'autre, un atome d'hydrogène, un atome d'halogène (F, Cl, Br, I), un groupe alkyle en C₁-C₁₂ saturé ou insaturé, un groupe alkyle en C₁-C₁₂ substitué par un atome d'halogène (F, Cl, Br, I), un groupe hydroxyalkyle (C₁-C₁₂), un groupe alcoxy en C₁-C₁₂, un groupe cyano, un groupe nitro, un groupe amino, un groupe alkyl(C₁-C₁₂)amino, un groupe dialkyl-(C₁-C₁₂)amino, un acide carboxylique, un groupe C(O)O-alkyle (C₁-C₁₂), un groupe C(O)O-phényle substitué ou non substitué, un groupe phényle substitué ou non substitué ou un groupe naphtyle, ou **R2** et **R3** forment ensemble avec la molécule restante un système cyclique hétérocyclique ou carbocyclique, saturé ou insaturé, substitué ou non substitué ;
(b) au moins un coupleur connu en soi ou un sel physiologiquement acceptable de celui-ci,
ainsi que (c) un persulfate en tant qu'agent oxydant.

2. Composition selon la revendication 1, **caractérisée en ce que X** est un atome de soufre, **A** est un atome d'hydrogène, **R1** est un groupe alkyle en C₁-C₁₂ saturé ou insaturé, un groupe hydroxyalkyle-(C₁-C₁₂), un groupe aminoalkyle(C₁-C₁₂) ou un groupe phényle substitué ou non substitué et **R2** et **R3** représentent, indépendamment l'un de l'autre, un atome d'hydrogène, un groupe alkyle en C₁-C₁₂ saturé ou insaturé, un groupe cyano, un groupe nitro, un groupe amino, un groupe alkyl(C₁-C₁₂) amino, un groupe dialkyl(C₁-C₁₂)amino, un groupe C(O)O-alkyle ou un groupe phényle substitué ou non substitué ou un groupe naphtyle, ou **R2** et **R3** forment ensemble avec la molécule restante un système cyclique carbocyclique, insaturé, substitué ou non substitué.

3. Composition selon la revendication 1 ou 2, **caractérisée en ce que** le dérivé d'hydrazone de formule (I) est choisi parmi la 3-méthyl-2(3H)-thiazolone-hydrazone, la 3,4-diméthyl-2(3H)-thiazolone-hydrazone, la 4-tert-butyl-3-méthyl-2(3H)-thiazolone-hydrazone, la 3-méthyl-4-phényl-2(3H)-thiazolone-hydrazone, la 3-méthyl-4-(4-tolyl)-2(3H)-thiazolone-hydrazone, la 4-(4-méthoxy)phényl-3-méthyl-2(3H)-thiazolone-hydrazone, la 4-(4-éthoxy)phényl-3-méthyl-2(3H)-thiazolone-hydrazone, la 4-(4-bromophényl)-3-méthyl-2(3H)-thiazolone-hydrazone, la 4-(3-bromophényl)-3-méthyl-2(3H~thiazolone-hydrazone, la 4-(4-chlorophényl)-3-méthyl-2(3H)-thiazolone-hydrazone, la 4-(3-chlorophényl)-3-méthyl-2(3H)-thiazolone-hydrazone, la 3-méthyl-4-(4-nitro-phényl)-2(3H)-thiazolone-hydrazone, la 3-méthyl-4-(3-nitrophényl)-2(3H)-thiazolone-hydrazone, la 4-([1,1'-biphényl]-4-yl)-3-méthyl-2(3H)-thiazolone-hydrazone, la 3-méthyl-4-(2-naphtalényl)-2(3H)-thiazolone-hydrazone, le 2-hydrazono-2,3-dihydro-3-méthyl-4-thiazolecarboxylate d'éthyle, la 3,4,5-triméthyl-2(3H)-thiazolone-hydrazone, la 3,4-diméthyl-5-phényl-2(3H)-thiazolone-hydrazone, la 3,5-diméthyl-4-phényl-2(3H)-thiazolone-hydrazone, la 3-méthyl-4,5-diphényl-2(3H)-thiazolone-hydrazone, la 5-éthyl-3-méthyl-4-phényl-2(3H)-thiazolone-hydrazone, la 4-(4-bromophényl)-3-méthyl-5-phényl-2(3H)-thiazolone-hydrazone, la 3-méthyl-5-phényl-4-(4-tolyl)-2(3H)-thiazolone-hydrazone, la 5-(4-chlorophényl)-4-phényl-3-méthyl-2(3H)-thiazolone-hydrazone, la 5-(4-chlorophényl)-4-(4-méthoxyphényl)-3-méthyl-2(3H)-thiazolone-hydrazone, le 2-hydrazono-2,3-dihydro-3,4-diméthyl-4-thiazolecarboxylate d'éthyle, le 4-amino-2-hydrazono-2,3-dihydro-3-méthyl-5-thiazole-carbonitrile, la 3-éthyl-4,5-diméthyl-2(3H)-thiazolone-hydrazone, le 2-hydrazono-2,3-dihydro-3-éthyl-4-méthyl-thiazolecarboxylate d'éthyle, la 5-méthyl-3-(1-mëthyléthyl)-4-phényl-2(3H)-thiazolone-hydrazone, la 4,5-diméthyl-3-(1-méthyléthyl)-2(3H)-thiazolone-hydrazone, la 3-(1-méthyléthyl)-4,5-diphényl-2(3H)-thiazolone-hydrazone, la 4,5-diméthyl-3-propyl-2(3H)-thiazolone-hydrazone, la 4,5-diphényl-3-propyl-2(3H)-thiazolone-hydrazone, la 3-butyl-4,5-diphényl-2(3H)-thiazolone-hydrazone, la 4,5-diméthyl-3-(2-méthylpropyl)-2(3H)-thiazolone-hydrazone, la 3-(2-méthyl-propyl)-4,5-diphényl-2(3H)-thiazolone-hydrazone, la 3-hydroxyéthyl-2(3H)-thiazolone-hydrazone, la 3-hydroxyéthyl-4-méthyl-2(3H)-thiazolone-hydrazone, la 3-hydroxy-éthyl-4,5-diméthyl-2(3H)-thiazolone-hydrazone, la 3-aminoéthyl-2(3H)-thiazolone-hydrazone, la 3-aminoéthyl-4-méthyl-2(3H)-thiazolone-hydrazone, la 3-aminoéthyl-4,5-diméthyl-2(3H)-thiazolone-hydrazone, la 3,4-diphényl-2(3H)-thiazolone-hydrazone, la 4-méthyl-3-phényl-2(3H)-thiazolone-hydrazone, la 4-p-biphénylyl-3-phényl-2(3H)-thiazolone-hydrazone, la 4-(4-méthoxy)phényl-3-phényl-2(3H)-thiazolone-hydrazone, la 4-tert-butyl-3-phényl-2(3H)-thiazolone-hydrazone, la 4,5-diméthyl-3-phényl-2(3H)-thiazolone-hydrazone, la 5-méthyl-3,4-diphényl-2(3H)-thiazolone-hydrazone, la 3,4,5-triphényl-2(3H)-thiazolone-hydrazone, la 4,5-diméthyl-3-(phénylméthyl)-2(3H)-thiazolone-hydrazone, la 3-(2-propényl)-2(3H)-thiazolone-hydrazone, la 4-méthyl-3-(2-propényl-2(3H)-thiazolone-hydrazone, la 4-tert-butyl-3-(2-propényl)-2(3H)-thiazolone-hydrazone, la 4-phényl-3-(2-propényl)-2(3H)-thiazolone-hydrazone, la 4,5-diméthyl-3-(2-propényl)-2(3H)-thiazolone-hydrazone, la 4,5-diphényl-3-(2-propényl)-2(3H)-thiazolone-hydrazone, le 2-hydrazono-2,3-dihydro-3-[(phénylamino)carbonyl]-4-méthylthiazole-carboxylate d'éthyle, la 3-méthyl-4,5,6,7-tétrahydro-2(3H)-benzothiazolone-hydrazone, la 3-méthyl-2(3H)-benzothiazolone-hydrazone, la 3,6-diméthyl-2(3H)-benzothiazolone-hydrazone, la 6-chloro-3-méthyl-2(3H)-benzothiazolone-hydrazone, la 7-chloro-3-méthyl-2(3H)-benzothiazolone-hydrazone, la 6-hydroxy-3-méthyl-2(3H)-benzothiazolone-hydrazone, la 5-méthoxy-3-méthyl-2(3H)-benzothiazolone-hydrazone, la 7-méthoxy-3-méthyl-2(3H)-benzothiazolone-hydrazone, la 5,6-diméthoxy-3-méthyl-2(3H)-benzothiazolone-hydrazone, la 5-éthoxy-3-méthyl-2(3H)-benzothiazolone-hydrazone, la 6-éthoxy-3-méthyl-2(3H)-benzothiazolone-hydrazone, la 3-méthyl-5-nitro-2(3H)-benzothiazolone-hydrazone, la 3-méthyl-6-nitro-2(3H)-benzothiazolone-hydrazone, la 5-acétamido-3-méthyl-2(3H)-benzothiazolone-hydrazone, la 6-acétamido-3-méthyl-2(3H)-benzothiazolone-hydrazone, la 5-anilino-3-méthyl-2(3H)-benzothiazolone-hydrazone, la 6-anilino-3-méthyl-2(3H)-benzothiazolone-hydrazone, l'acide 2-hydrazono-2,3-dihydro-3-méthyl-6-benzothiazole-carboxylique, l'acide 2-hydrazono-2,3-dihydro-3-méthyl-4-benzothiazole-sulfonique, l'acide 2-hydrazono-2,3-dihydro-3-méthyl-5-benzothiazole-sulfonique, l'acide 2-hydrazono-2,3-dihydro-3-méthyl-6-benzothiazole-sulfonique, l'acide 2-hydrazono-2,3-dihydro-3-méthyl-7-benzothiazole-sulfonique, le 2-hydrazono-2,3-dihydro-N,N,3-triméthyl-6-benzothiazole-sulfonamide, le [(2-hydrazono-2,3-dihydro-3-méthyl-6-benzothiazolyl)-oxy] acéto-hydrazide, la 3-méthyl-naphto [2, 3-d] - thiazol-2(3H)-one-hydrazone, la 3-éthyl-2(3H)-benzothiazolone-hydrazone, la 6-éthoxy-3-éthyl-2(3H)-benzothiazolone-hydrazone, la 3-propyl-2(3H)-benzothiazolone-hydrazone, la 3-butyl-2(3H)-benzothiazolone-hydrazone, la 3-hexyl-2(3H)-benzothiazolone-hydrazone, la 3-hydroxyéthyl-2(3H)-benzothiazolone-hydrazone, la 3-aminoéthyl-2(3H)-benzothiazolone-hydrazone, la 3-p-méthyl-benzyl-2(3H)-benzothiazolone-hydrazone, l'acide 2-hydrazono-2,3-dihydro-3-(2-hydroxyéthyl)-6-benzothiazole-carboxylique, l'acide 2-hydrazono-2,3-dihydro-6-méthoxy-3(2H)benzothiazole-propane-sulfonique, l'acide 6-hexadécyloxy-2-hydrazono-3(2H)-benzothiazole-propanesulfonique, la 2-oxo-3-benzothiazoline-acétate d'éthyle-hydrazone, la 3-acetyl-2(3H)-benzothiazolone-hydrazone, la 2-hydrazono-3(2H)-benzothiazole-carboxaldéhyde, la 3-méthyl-2(3H)-oxazolone-hydrazone, la 3-phényl-2(3H)-oxazolone-hydrazone, la 3-méthyl-2(3H)-benzoxazolone-hydrazone, la 3-phényl-2(3H)-benzoxazolone-hydrazone, la N-acétyl-3-méthyl-2(3H)-thiazolone-hydrazone, la N-acétyl-3,4-diméthyl-2(3H)-thiazolone-hydrazone, la N-acétyl-3-méthyl-4-phényl-2(3H)-thiazolone-hydrazone, la N-acétyl-4-(4-méthoxy)phényl-3-méthyl-2(3H)-thiazolone-hydrazone, la N-acétyl-3-méthyl-4-(4-nitrophényl)-2(3H)-thiazolone-hydrazone, N-acetyl-4-([1,1'-biphényl]-4-yl)-3-méthyl-2(3H)-thiazolone-hydrazone, la N-acétyl-3-méthyl-4-(2-naphtalényl)-2(3H)-thiazolone-hydrazone, le N-acétyl-2-hydrazono-2,3-dihydro-3-méthyl-4-thiazolecarboxylate d'éthylester, la N-acétyl-3,4,5-triméthyl-2(3H)-thiazolone-hydrazone, la N-acétyl-3,4-diméthyl-5-phényl-2(3H)-thiazolone-hydrazone, la N-acétyl-3,5-diméthyl-4-phényl-2(3H)-thiazolone-hydrazone, la N-acétyl-3-méthyl-4,5-diphényl-2(3H)-thiazolone-hydrazone, la N-acetyl-3-éthyl-4,5-diméthyl-2(3H)-thiazolone-hydrazone, la N-acétyl-4-méthyl-3-phényl-2(3H)-thiazolone-hydrazone, la N-acétyl-3,4-diphényl-2(3H)-thiazolone-hydrazone, la N-acétyl-4-p-biphénylyl-3-phényl-2(3H)-thiazolone-hydrazone, la N-acétyl-4-(4-méthoxy)phényl-3-phényl-2(3H)-thiazolone-hydrazone, la N-acétyl-4-tert-butyl-3-phényl-2(3H)-thiazolone-hydrazone, la N-acétyl-4,5-diméthyl-3-phényl-2(3H)-thiazolone-hydrazone, la N-acétyl-5-méthyl-3,4-diphényl-2(3H)-thiazolone-hydrazone, la N-acétyl-3,4,5-triphényl-2(3H)-thiazolone-hydrazone, la N-acétyl-3-méthyl-2(3H)-benzothiazolone-hydrazone, la N-acétyl-3-éthyl-2(3H)-benzothiazolone-hydrazone, la N-acétyl-3-butyl-2(3H)-benzothiazolone-hydrazone, la N-acétyl-3-hexyl-2(3H)-benzothiazolone-hydrazone, la N-acétyl-3-p-méthylbenzyl-2(3H)-benzothiazolone-hydrazone, la N-acétyl-3-méthyl-2(3H)-oxazolone-hydrazone, la N-acétyl-3-phényl-2(3H)-oxazolone-hydrazone, la N-acétyl-3-méthyl-2(3H)-benzoxazolone-hydrazone, la N-acétyl-3-phényl-2(3H)-benzoxazolone-hydrazone, la N-formyl-3-méthyl-2(3H)-thiazolone-hydrazone, la N-formyl-3,4-diméthyl-2(3H)-thiazolone-hydrazone, la N-formyl-3-méthyl-4-phényl-2(3H)-thiazolone-hydrazone, la N-formyl-4-(4-méthoxy)phényl-3-méthyl-2(3H)-thiazolone-hydrazone, la N-formyl-3-méthyl-4-(4-nitrophényl)-2(3H)-thiazolone-hydrazone, la N-formyl-4-([1,1'-biphényl]-4-yl)-3-méthyl-2(3H)-thiazolone-hydrazone, la N-formyl-3-méthyl-4-(2-naphtalényl)-2(3H)-thiazolone-hydrazone, le N-formyl-2-hydrazono-2,3-dihydro-3-méthyl-4-thiazolecarboxylate d'éthyle, la N-formyl-3,4,5-triméthyl-2(3H)-thiazolone-hydrazone, la N-formyl-3,4-diméthyl-5-phênyl-2(3H)-thiazolone-hydrazone, la N-formyl-3,5-diméthyl-4-phényl-2(3H)-thiazolone-hydrazone, N-formyl-3-méthyl-4,5-diphényl-2(3H)thiazolone-hydrazone, la N-formyl-3-éthyl-4,5-diméthyl-2(3H)-thiazolone-hydrazone, la N-formyl-4-méthyl-3-phényl-2(3H)-thiazolone-hydrazone, la N-formyl-3,4-diphényl-2(3H)-thiazolone-hydrazone, la N-formyl-4-p-biphénylyl-3-phényl-2(3H)-thiazolone-hydrazone, la N-formyl-4-(4-méthoxy)phényl-3-phényl-2(3H)-thiazolone-hydrazone, la N-formyl-4-tert-butyl-3-phényl-2(3H)-thiazolone-hydrazone, la N-formyl-4,5-diméthyl-3-phényl-2(3H)-thiazolone-hydrazone, la N-formyl-5-méthyl-3,4-diphényl-2(3H)-thiazolone-hydrazone, la N-formyl-3,4,5-triphényl-2(3H)-thiazolone-hydrazone, la N-formyl-3-méthyl-2(3H)-benzothiazolone-hydrazone, la N-formyl-3-éthyl-2(3H)-benzothiazolone-hydrazone, la N-formyl-3-butyl-2(3H)-benzothiazolone-hydrazone, la N-formyl-3-hexyl-2(3H)-benzothiazolone-hydrazone, la N-formyl-3-p-méthylbenzyl-2(3H)-benzothiazolone-hydrazone, la N-formyl-3-méthyl-2(3H)-oxazolone-hydrazone, la N-formyl-3-phényl-2(3H)-oxazolone-hydrazone, la N-formyl-3-méthyl-2(3H)-benzoxazolone-hydrazone et la N-formyl-3-phényl-2(3H)-benzoxazolone-hydrazone.

4. Composition selon l'une quelconque des revendications 1 à 3, **caractérisée en ce que** le coupleur est choisi parmi la N-(3-diméthylaminophényl)urée, la 2,6-diaminopyridine, le 2-amino-4-[(2-hydroxyéthyl)amino]anisole, le 2,4-diamino-1-fluoro-5-méthylbenzène, le 2,4-diamino-1-méthoxy-5-méthylbenzène, le 2,4-diamino-1-éthoxy-5-méthylbenzène, le 2,4-diamino-1-(2-hydroxyéthoxy)-5-méthylbenzène, le 2,4-di[(2-hydroxyéthyl)amino]-1,5-diméthoxybenzène, la 2,3-diamino-6-méthoxypyridine, la 3-amino-6-méthoxy-2-(méthylamino)pyridine, la 2,6-diamino-3,5-diméthoxypyridine, la 3,5-diamino-2,6-diméthoxypyridine, le 1,3-diaminobenzène, le 2,4-diamino-1-(2-hydroxyéthoxy)benzène, le 1,3-diamino-4-(2,3-dihydroxypropoxy)benzène, le 1,3-diamino-4-(3-hydroxypropoxy)benzène, le 1,3-diamino-4-(2-méthoxyéthoxy)benzène, le 2,4-diamino-1,5-di(2-hydroxyéthoxy)benzène, le 1-(2-aminoéthoxy)-2,4-diaminobenzène, le 2-amino-1-(2-hydroxyéthoxy)-4-méthylaminobenzène, l'acide 2,4-diaminophénoxyacétique, la 3-[di(2-hydroxyéthyl)-amino]aniline, le 4-amino-2-di[(2-hydroxyéthyl)-amino]-1-éthoxybenzène, le 5-méthyl-2-(1-méthyl-éthyl)phénol, la 3-[(2-hydroxyéthyl)amino]aniline, la 3-[(2-aminoéthyl)amino]aniline, le 1,3-di(2,4-diaminophénoxy)propane, le di(2,4-diaminophénoxy)-méthane, le 1,3-diamino-2,4-diméthoxybenzène, le 2,6-bis(2-hydroxyéthyl)aminotoluène, le 4-hydroxyindole, le 3-diméthylaminophénol, le 3-diéthyl-aminophénol, le 5-amino-2-méthylphénol, le 5-amino-4-fluoro-2-méthylphénol, le 5-amino-4-méthoxy-2-méthylphénol, le 5-amino-4-éthoxy-2-méthylphénol, le 3-amino-2,4-dichlorophénol, le 5-amino-2,4-dichlorophénol, le 3-amino-2-méthyl-phénol, le 3-amino-2-chloro-6-méthylphénol, le 3-aminophénol, le 2-[(3-hydroxyphényl)amino]-acétamide, le 5-[(2-hydroxyéthyl)amino]-4-méthoxy-2-méthylphénol, le 5-[(2-hydroxyéthyl)amino]-2-méthylphénol, le 3-[(2-hydroxyéthyl)amino]phénol, 3-[(2-méthoxyéthyl)amino]phénol, le 5-amino-2-éthylphénol, le 5-amono-2-méthoxyphénol, le 2-(4-amino-2-hydroxyphénoxy)éthanol, le 5-[(3-hydroxy-propyl)amino]-2-méthylphénol, le 3-[(2,3-dihydroxypropyl)amino]-2-méthylphénol, le 3-[(2-hydroxyéthyl)amino]-2-méthyl-phénol, la 2-amino-3-hydroxypyridine, la 2,6-dihydroxy-3,4-diméthyl-pyridine, le 5-amino-4-chloro-2-méthylphénol, le 1-naphtol, le 2-méthyl-1-naphtol, le 1,5-dihydroxynaphthalène, le 1,7-dihydroxynaphthalène, le 2,3-dihydroxynaphthalène, le 2,7-dihydroxynaphthalène, l'acétate de 2-méthyl-1-naphtol, le 1,3-dihydroxybenzène, le 1-chloro-2,4-dihydroxybenzène, le 2-chloro-1,3-dihydroxybenzène, 1,2-dichloro-3,5-dihydroxy-4-méthylbenzène, le 1,5-dichloro-2,4-dihydroxybenzène, le 1,3-dihydroxy-2-méthylbenzène, le 3,4-méthylènedioxyphénol, la 3,4-méthylènedioxyaniline, le 5-[(2-hydroxyéthyl)-amino]-1,3-benzodioxole, le 6-bromo-1-hydroxy-3,4-méthylènedioxybenzène, l'acide 3,4-diamino-benzoïque, la 3,4-dihydro-6-hydroxy-1,4(2H)-benzoxazine, la 6-amino-3,4-dihydro-1,4(2H)-benzoxazine, la 3-méthyl-1-phényl-5-pyrazolone, le 5,6-dihydroxyindole, la 5,6-dihydroxyindoline, le 5-hydroxyindole, le 6-hydroxyindole, le 7-hydroxyindole et la 2,3-indolinedione.

5. Composition selon l'une quelconque des revendications 1 à 4, **caractérisée en ce que** le persulfate est choisi parmi le persulfate de potassium, le persulfate de sodium et le persulfate d'ammonium.

6. Composition selon l'une quelconque des revendications 1 à 5, **caractérisée en ce qu'**elle contient les dérivés d'hydrazone de formule (I) ainsi que les coupleurs et les persulfates chacun en une quantité totale de 0,01 à 10% en poids.

7. Composition selon l'une quelconque des revendications 1 à 5, **caractérisée en ce qu'**elle contient en outre de 0,01 à 10% en poids d'un colorant direct physioloiquement sans inconvénient, choisi dans le groupe des colorants cationiques et des colorants anioniques, des colorants en dispersion, des colorants nitrés, des colorants azoïques, des colorants quinoniques et des colorants triphénylméthane.

8. Composition selon l'une quelconque des revendications 1 à 7, **caractérisée en ce qu'**elle présente un pH de 3 à 10.

9. Composition selon l'une quelconque des revendications 1 à 8, **caractérisée en ce qu'**elle est une composition de teinture pour cheveux.

10. Nécessaire à 2 composants pour la teinture de fibres de kératine, constitué d'une matière colorante (A1) qui contient le composé de formule (I), et d'une autre matière colorante (A2) qui contient les coupleurs et les persulfates, ainsi qu'éventuellement d'un agent pour l'ajustement du pH.

11. Nécessaire à 2 composants pour la teinture de fibres de kératine, dont le premier composant consiste en une poudre contenant les composés de formule (I), les coupleurs et les persulfates ainsi qu'éventuellement d'autres adhésifs cosmétiques pulvérulents usuels, et dont le deuxième composant est de l'eau ou une préparation cosmétique liquide, qui contient éventuellement un agent pour l'ajustement du pH.

12. Procédé pour la teinture des cheveux, dans lequel on applique sur les cheveux une composition de teinture selon l'une quelconque des revendications 1 à 9 et, après un temps d'action de 5 à 60 minutes à une température de 20 à 50 °C, on rince les cheveux à l'eau, éventuellement on les lave avec un shampooing et ensuite on les sèche.

13. Procédé pour la teinture des cheveux, dans lequel on prépare immédiatement avant l'emploi la composition de teinture (A) prête à l'emploi, par mélange de deux composants (A1) et (A2) - éventuellement avec addition d'un agent d'alcalinisation ou d'un acide - et ensuite on l'applique sur les cheveux et, après un temps d'action de 5 à 60 minutes à une température de 20 à 50 °C, on rince les cheveux à l'eau, éventuellement on les lave avec un shampooing et ensuite on les sèche, **caractérisé en ce qu'**on utilise une composition de teinture (A) selon la revendication 10 ou 11, pouvant être obtenue par mélange de deux composants (A1) et (A2).
